(19) Europäisches Patentamt
European Patent Office
Office européen des brevets

(11) EP 3 374 524 B1

(12) **EUROPEAN PATENT SPECIFICATION**

(45) Date of publication and mention of the grant of the patent:
**29.01.2025 Bulletin 2025/05**

(21) Application number: **16863787.4**

(22) Date of filing: **10.11.2016**

(51) International Patent Classification (IPC):
***C12Q 1/6886*** *(2018.01)* ***C12Q 1/48*** *(2006.01)*
***G01N 33/58*** *(2006.01)* ***G01N 33/52*** *(2006.01)*
***C12Q 1/6806*** *(2018.01)*

(52) Cooperative Patent Classification (CPC):
(C-Sets available)
**C12Q 1/6886; C12Q 1/6806;** C12Q 2600/154
(Cont.)

(86) International application number:
**PCT/IL2016/051218**

(87) International publication number:
**WO 2017/081689 (18.05.2017 Gazette 2017/20)**

(54) **METHODS OF DETECTING 5-HYDROXYMETHYLCYTOSINE AND DIAGNOSING OF CANCER**

VERFAHREN ZUM NACHWEIS VON 5-HYDROXYMETHYLCYTOSIN UND ZUR DIAGNOSE VON KREBS

PROCÉDÉS DE DÉTECTION DE LA 5-HYDROXYMÉTHYLCYTOSINE ET DIAGNOSTIC DU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.11.2015 US 201562253797 P**

(43) Date of publication of application:
**19.09.2018 Bulletin 2018/38**

(73) Proprietor: **Ramot at Tel-Aviv University Ltd.**
**6139201 Tel-Aviv (IL)**

(72) Inventors:
- **EBENSTEIN, Yuval**
**8150169 Yavne (IL)**
- **ZIRKIN, Shahar**
**6139201 Tel-Aviv (IL)**
- **MICHAELI HOCH, Yael**
**4252480 Netanya (IL)**

(74) Representative: **Boult Wade Tennant LLP**
**Salisbury Square House**
**8 Salisbury Square**
**London EC4Y 8AP (GB)**

(56) References cited:
**WO-A1-2011/127136 WO-A1-2013/188436**
**WO-A1-2014/152279 WO-A1-2014/191981**
**US-A1- 2014 030 727 US-A1- 2014 038 183**
**US-A1- 2014 080 715**

- **NOA GILAT ET AL: "Single-molecule quantification of 5-hydroxymethylcytosine for diagnosis of blood and colon cancers", CLINICAL EPIGENETICS, BIOMED CENTRAL LTD, LONDON, UK, vol. 9, no. 1, 14 July 2017 (2017-07-14), pages 1 - 8, XP021247144, ISSN: 1868-7075, DOI: 10.1186/S13148-017-0368-9**
- **GIL NIFKER ET AL: "One-Pot Chemoenzymatic Cascade for Labeling of the Epigenetic Marker 5-Hydroxymethylcytosine", CHEMBIOCHEM, vol. 16, no. 13, 27 July 2015 (2015-07-27), pages 1857 - 1860, XP055589051, ISSN: 1439-4227, DOI: 10.1002/cbic.201500329**
- **TAMAR SHAHAL ET AL: "Spectroscopic Quantification of 5-Hydroxymethylcytosine in Genomic DNA", ANALYTICAL CHEMISTRY, vol. 86, no. 16, 29 July 2014 (2014-07-29), US, pages 8231 - 8237, XP055589033, ISSN: 0003-2700, DOI: 10.1021/ac501609d**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **YAEL MICHAELI ET AL: "Optical detection of epigenetic marks: sensitive quantification and direct imaging of individual hydroxymethylcytosine bases", CHEMICAL COMMUNICATIONS, vol. 49, no. 77, 7 October 2013 (2013-10-07), pages 8599 - 8720, XP055589031, ISSN: 1359-7345, DOI: 10.1039/ c3cc42543f**

(52) Cooperative Patent Classification (CPC): (Cont.)

C-Sets
**C12Q 1/6806, C12Q 2537/164, C12Q 2563/107, C12Q 2565/601**

## Description

The project leading to this application has received funding from the European Union's Horizon 2020 research and innovation program under grant agreement No. 634890.

## FIELD AND BACKGROUND OF THE INVENTION

The present invention, in some embodiments thereof, relates to methods of detecting and quantifying the level of 5-hydroxymethylcytosine.

Detection of cancer at an early stage is critical for successful treatment and increasing survivability. Cancer arises due to the accumulation of DNA alterations that result in cells that uncontrollably proliferate. A common DNA alteration (> 95 %) is cytosine methylation. Cytosine methylation is an epigenetic modification which is catalyzed by DNA cytosine-5methyltransferases (DNMTs) and occurs at the 5-position (C5) of the cytosine ring, within CpG dinucleotides. Global 5methylcytosine (5mC) in cancer cells is generally reduced compared to that in normal cells. Decrease in global 5mC or DNA hypomethylation is likely caused by methyl-deficiency due to a variety of environmental influences, and has been proposed as a molecular marker in cancer. It is well demonstrated that the decrease in global 5mC is one of the most important characteristics of cancer [Feinberg A P et al., Nature, 1983 301, 89-92]. Determination of global 5mC contents has been used as a biomarker to dehne diagnostic potential in several types of cancers. However, the decrease in the level of global 5mC in cancer cells is not significant compared to that measured in normal cells, which cannot allow to sufficiently discriminate cancer states from normal states in a sample tested, as 5mC content in cancer tissues is only 10-20% lower than that in normal tissues [Finberg A P et al., Cancer Res, 1988 48:159]. 5-hydroxymethylcytosine (5-hmc), which is hydroxylated and methylated form of cytosine has been detected to be abundant in mouse brain and embryonic stem cells [Kriaucionis S et al.: Science, 2009]. In mammals, it can be generated by oxidation of 5mC, a reaction mediated by the Tet family of enzymes [Tahiliani M et al.: Science, 2009]. The function of 5-hmc in epigenetics is still unclear. However, a line of evidence showed that 5-hmc plays a role in DNA demethylation, chromatin remodeling and gene expression regulation in a tissue-, cell- or organ-specific manner [Valinluck V et al.: Cancer Res, 2007, Valinluck V et al.: Nucleic Acid res, 2004, Penn N W et al.: Biochem J, 1976, Penn N W et al.: biochem J, 1972]. 5-hmc may also negatively regulate cancer formation and development. Numerous evidence showed that methylation-mediated silencing of tumor suppression and apoptosis genes is involved in cancer formation and progression. 5-hmc has been demonstrated to facilitate passive DNA demethylation by blocking the maintenance DNA methyltransferase DNMT1 to methylate DNA containing 5-hmc [Valinluck V et al., Nucleic Acids Res 2004; Liutkeviciute Z et al., Nat Chem Biol 2009] and to participate in active DNA demethylation by enzymatic or spontaneous conversion of 5mC [Tahiliani M et al. Science 2009; Ito S et al., Nature, 2010]. Thus, it is possible that the 5-hmc could affect the reactivation of these genes through 5-hmc-mediated demethylation and help to restore the tumor suppression and apoptosis function of these genes. 5-hmc levels in cancer tissues are significantly reduced compared to their normal counterparts, suggesting a complex and yet to be defined role of 5-hmc in tissue differentiation and neoplasia. 5-hmC is 10 - 1000 times less abundant than 5-mC and therefore is difficult to quantify in some tissues. Due to the general decrease in 5-hmc level in cancer, it is impossible to detect its levels in in-situ biopsies, let alone in the blond, using clinically relevant assays.

WO 2014/191981 A1 and Gil Nifker et al.: "One-Pot Chemoenzymatic Cascade for Labeling of the Epigenetic Marker 5-Hydroxymethylcytosine", CHEMBIOCHEM, vol. 16, no. 13, 27 July 2015 (2015-07-27), pages 1857-1860, XP055589051, ISSN: 1439-4227, DOI: 10.1002/cbic.201500329 both show a method for detecting the level of 5-hmc in genomic DNA, while US 2014/030727 A1 discloses a method for detecting or diagnosing cancer by measuring levels of 5-hmc.

Various methods for the detection of 5-hmc in genomic DNA are known, as also shown in TAMAR SHAHAL ET AL: "Spectroscopic Quantification of 5-Hydroxymethylcytosine in Genomic DNA", ANALYTICAL CHEMISTRY, vol. 86, no. 16, 29 July 2014 (2014-07-29), pages 8231-8237, XP055589033, US ISSN: 0003-2700, DOI: 10.102 1/a c501609d; YAEL MICHAELI ET AL: "Optical detection of epigenetic marks: sensitive quantification and direct imaging of individual hydroxymethylcytosine bases", CHEMICAL COMMUNICATIONS, vol. 49, no. 77, 7 October 2013 (2013-10-07), pages 8599-8720, XP055589031, ISSN: 1359-7345, DOI: 10.1039/c3cc42543f and WO 2011/127136 Al (UNIV CHICAGO [US]; HE CHUAN [US]; SONG CHUNXIAO [US]) 13 October 2011 (2011-10-13). However, at low levels of 5-hmc the measurement can become inaccurate by use of these methods.

Additional background art includes:

Haffner et al. Oncotarget 2011 2:627-637
U.S. Application Number 20120122087
Song et al. 2011 Nat. Biotech. 29:68-72
Ko et al. Immunological Reviews 263:6-21
Chen et al. Clinical Chemistry 2013 59:5

## SUMMARY OF THE INVENTION

According to an aspect of some embodiments of the present invention there is provided a method for detecting and quantifying the level of 5-hydroxymethylcytosine (5-hmc) in a cell-free DNA molecule of a subject

using an imaging method, the method comprising: associating a label with the 5-hydroxylmethylcytosine, wherein the label comprises a

fluorescent label, attaching to the DNA molecule an additional labelling agent distinct of the 5-hmc specific label, wherein the additional labelling agent is a non-specific fluorescent label for determining overall DNA fluorescence intensity, and quantifying the ratio between overall DNA fluorescence intensity and overall 5hmC fluorescence intensity, thereby determining the level of 5-hmc in the cell-free DNA molecule, wherein the method is capable of detecting the level of 5-hmc in a DNA molecule of a cell having a 5-hmc prevalence lower than 0.002 % of total DNA.

(a)

According to some embodiments of the invention, the cell is a cell line.

According to some embodiments of the invention, the cell is a primary cell.

According to an aspect of some embodiments of the present invention, there is provided a method of diagnosing cancer in a subject in need thereof, the method comprising:

(a) providing a DNA sample of a cell of the subject;

(b) detecting the level of 5-hmc in the DNA sample according to the method of claim 1; wherein a significant decrease in the level of 5-hmc in the DNA sample, as compared to a control DNA sample from a healthy subject is indicative that the subject has cancer.

According to some embodiments of the invention, the significant decrease is below 50 %.

According to some embodiments of the invention, the cell has a 5-hmc prevalence lower than 0.01%.

According to some embodiments of the invention, the cell has a 5-hmc prevalence lower than 0.005%.

According to some embodiments of the invention, the cell has a 5-hmc prevalence lower than 0.004%.

According to some embodiments of the invention, the cell has a 5-hmcprevalence lower than 0.003%.

According to some embodiments of the invention, the cancer is a soft tissue cancer.

According to some embodiments of the invention, the soft tissue cancer is selected from the group consisting of leukemia and multiple myeloma.

According to some embodiments of the invention, the cancer is a solid tumor.

According to some embodiments of the invention, the cancer is of the gastrointestinal system (GI).

According to some embodiments of the invention, the cancer of the GI system is selected from the group consisting of colon cancer and rectal cancer.

According to some embodiments of the invention, the cancer is a soft tissue tumor or a solid tumor and the cell is a PBMC.

According to some embodiments of the invention, the cancer is a soft tissue tumor or a solid tumor and the cell is a lymphocyte.

According to some embodiments of the invention, the cancer is a solid tumor and the cell is of the tumor (in situ or metastasis).

According to some embodiments of the invention, attaching the labeling agent comprises:
reacting a labeling agent derivatized by a second reactive group with a DNA molecule in the DNA sample in which the 5-hydroxymethylcytosines are glycosylated by a glucose molecule derivatized by a first reactive group, wherein the first and second reactive groups are chemically compatible to one another.

According to some embodiments of the invention, glycosylating the 5hydroxymethylcytosines in the DNA molecule comprises incubating the DNA molecule with β-glucosyltransferase and a uridine diphosphoglucose (UDP-Glu) derivatized by the first reactive group.

According to some embodiments of the invention, one of the first and second reactive groups is azide and the other is alkyne, such that attaching the labeling agent to the DNA molecule is effected by a dick chemistry.

According to some embodiments of the invention, the reacting is free of a copper catalyst.

According to some embodiments of the invention, the first reactive group is azide.

According to some embodiments of the invention, the uridine diphosphoglucose (UDP-G1u) derivatized by the first reactive group is a UDP-6-$N_3$-Glucose.

According to some embodiments of the invention, the UDP-6-$N_3$-Glucose is synthesized chemically.

According to some embodiments of the invention, the UDP-6-$N_3$-Glucose is synthesized enzymatically.

According to some embodiments of the invention, the method further comprises extending DNA molecule(s) of the DNA sample prior to imaging.

According to some embodiments of the invention, the extending is linearly extending. According to some embodiments of the invention, the extending is effected following step (a).

According to some embodiments of the invention, the method does not comprise subjecting the DNA molecule(s) to fragmentation.

According to some embodiments of the invention, the extending is effected by depositing the DNA molecule(s) an a surface or extending the DNA molecule in a nanochannel.

According to some embodiments of the invention, the method further comprises identifying a position of the 5-hydroxymethyl-cytosine (5-hmc) along the DNA molecule(s).

According to the invention, the method comprises reacting the 5hmc-specific fluorescent agent under conditions which allow staining of the DNA molecule(s) with the 5hmc-specific labeling agent so as to obtain a 5hmC-labeled DNA sample; and measuring fluorescence intensity of the 5hmC-labeled DNA molecule(s) (X) and DNA stain intensity (Y), wherein a ratio between X to Y is indicative of presence or level of 5hmC in the DNA sample.

According to some embodiments of the invention, the cell is a cancer cell having a 5-hmc prevalence above 0.002 %.

According to some embodiments of the invention, the cancer cell is a solid tumor cancer.

According to some embodiments of the invention, the ratio is compared to a ratiometric calibration curve.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

## BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

Figure 1 shows the detection of 5-hmc in DNA extracted from a brain tissue (left) or a kidney tissue (middle). The DNA was stretched on a cover slip glass and visualized by fluorescent microscope. All pictured data was analyzed using a proprietary software that automatically processes the images and classifies the detected DNA molecules. The software counts the number of 5-hmc per length or intensity of the DNA and calculates the percentage of 5-hmc from total DNA bases (left).

Figure 2 shows the detection of 5-hmc level in Hela, HEK293 and U2OS cell lines, compared to PBMCs of healthy individuals. As low as 0.001 % or even lower levels of 5-hmc can be detected from any DNA sample tested.

Figure 3 shows global level of 5-hmc in blood of multiple myeloma and leukemia patients compared to healthy individuals in their peripheral blood. The level of 5-hmc is dropping on average of 25 % when comparing multiple myeloma patients to healthy individuals. The same results were achieved when comparing leukemia patients to healthy individuals.

Figure 4 shows DNA extracted from human PBMCs and stretched in nanochannel arrays (BioNano Genomics): DNA molecules in blue, red dots are genetic tags for mapping to the genome and green dots are 5hmC.

Figures 5A-B show 5-hmc levels in colorectal cancer, pre-malignant tissue and healthy control. (Figure 5A) Fluorescence microscopy images of representative DNA molecules with 5-hmC labeling: left panel - healthy colorectal, middle panel - colorectal tumor, right panel - adjacent tissue. (Figure 5B) The box plot compares 5-hmC level calculated from imaged molecules of healthy colorectal *(n=7)*, colorectal tumor *(n=7)* and adjacent tissue (n=7).

## DESCRIPTION OF SPECIFIC EMBODIMENTS OF THE INVENTION

The present invention, in some embodiments thereof, relates to methods of detecting and quantifying the level of 5-hydroxymethylcytosine.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways. 5-hydroxymethylcytosine (5-hmc) is a recently rediscovered epigenetic modification of DNA with tissue and cell type specific distribution in mammalian genomes. The level of 5-hmc in peripheral blood is estimated to be as low as 0.002 % from total DNA bases. This fact makes it impossible to detect via existing commercial methods due to limit of sensitivity.

Whilst reducing the present invention to practice, the present inventors were able to detect as low as 0.001 % of 5-hmc in peripheral blood and in various cell lines using optical mapping of DNA and detection of fluorescently labeled 5-hmC residues. Although it has been suggested that the global level of 5-hmc in genomic DNA is reduced in cancer tissues, it is still not clear what the 5-hmc level is in peripheral blood of cancer patients. It was found that using the method of some embodiments of the invention, it is possible to quantify small differences (as low as 5 % difference) in 5-hmc content between different samples such as peripheral blood samples. On average the 5-hmc level in peripheral blood of multiple myeloma and leukemia patients is 25% lower than compared to healthy individuals.

According to an aspect of the invention there is

provided a method for detecting and quantifying the level of 5-hydroxymethylcytosine (5-hmc) in a cell-free DNA molecule of a subject using an imaging method, the method comprising:

associating a label with the 5-hydroxylmethylcytosine, wherein the label comprises a fluorescent label and quantifying the ratio between overall DNA fluorescence intensity and overall 5hmC fluorescence intensity, thereby determining the level of 5-hmc in the cell-free DNA molecule, wherein the method is capable of detecting the level of 5-hmc in a DNA molecule of a cell having a 5-hmc prevalence lower than 0.002 % of total DNA.

According to a specific embodiment, cells having a 5-hmc prevalence lower than 0.0018 % of total DNA bases are selected according to the method.

According to a specific embodiment, cells having a 5-hmc prevalence lower than 0.0017 % of total DNA bases are selected according to the method.

According to a specific embodiment, cells having a 5-hmc prevalence lower than 0.0016 % of total DNA bases are selected according to the method.

According to a specific embodiment, cells having a 5-hmc prevalence lower than 0.0015 % of total DNA bases are selected according to the method.

According to a specific embodiment, cells having a 5-hmc prevalence lower than 0.0014 % of total DNA bases are selected according to the method.

According to a specific embodiment, cells having a 5-hmc prevalence lower than 0.0013 % of total DNA bases are selected according to the method.

According to a specific embodiment, cells having a 5-hmc prevalence lower than 0.0012 % of total DNA bases are selected according to the method.

According to a specific embodiment, cells having a 5-hmc prevalence lower than 0.0011 % of total DNA bases are selected according to the method.

According to a specific embodiment, cells having a 5-hmc prevalence lower than 0.0001 % of total DNA bases are selected according to the method (e.g., Figure 5). According to a specific embodiment, the cell is a cancer cell.

According to a specific embodiment, the cell is a pathogenic immune cell.

According to a specific embodiment, the cell is a cell line (e.g., cancer cell line).

According To a specific embodiment, the cell is a primary cell, e.g., non-cultured cancer cell or pathogenic immune cell.

According to a specific embodiment, the cell is a healthy cell e.g., used in screening populations, screening assays or as a control.

According to a specific embodiment, the cell is a pre-malignant tissue or lesion.

As used herein "pre-malignant" refers to a tissue that is not yet malignant but is poised to become malignant. Appropriate clinical and laboratory studies are designed to detect premalignant tissue while it is still in a premalignant stage. Examples of premalignant growths include polyps in the colon, actinic keratosis of the skin, dysplasia of the cervix, metaplasia of the lung, pre-malignant lesions of oral squamous cell carcinoma (OSCC) and leukoplakia (white patches in the mouth).

According to a specific embodiment, the pre-malignant tissue is of colorectal cancer (see e.g., Figures 5A-B).

According to a specific embodiment, the pre-malignant lesion which is diagnosed by the method of this aspect of the present invention is an adenomatous polyp of the colon, an adenomatous polyp of the rectum, an adenomatous polyp of the small bowel and Barrett's esophagus.

Typically, the pre-malignant lesion has a 5-hmc prevalence which is intermediate between that of a healthy tissue and that of a cancerous tissue (e.g., all data is available from the same subject).

According to a specific embodiment, the cancer is a soft tissue tumor or a solid tumor and the cell is an immune cell e.g., PBMC e.g., a lymphocyte.

According to an aspect of the invention , there is provided a method of diagnosing cancer in a subject in need thereof, the method comprising:

(a) providing a DNA sample of a cell of the subject;

(b) detecting the level of 5-hmc in the DNA sample according to the method described herein; wherein a significant decrease in the level of 5-hmc in the DNA sample, as compared to a control DNA sample from a healthy subject is indicative that the subject has cancer.

As used herein providing a DNA sample of a cell of a subject, refers to a tissue biopsy.

The biopsy can be taken from a non-affected/-suspected region (e.g., control), a region diagnosed with cancer and/or a region suspected of being cancerous or premalignant and that can be in the vicinity of a cancerous/affected region.

As used herein "significant decrease" refers to a decrease that is statistically significant (e.g., $P<0.05$).

Typically, the decrease is subtle between the normal control and the pathogenic sample and therefore the sensitivity of the method of 5-hmc detection is crucial. This is further emphasized in pre-malignant stages, as shown in

According to a specific embodiment, the significant decrease is below 50 %.

According to a specific embodiment, the significant decrease is between 5-45%.

According to a specific embodiment, the significant decrease is between 10-50 %.

According to a specific embodiment, the significant decrease is between 10-45%.

According to a specific embodiment, the significant decrease is between 20-50%.

According to a specific embodiment, the significant decrease is between 20-45%.

According to a specific embodiment, the significant decrease is between 30-50%.

According to a specific embodiment, the significant decrease is between 30-45%.

According to a specific embodiment, the significant decrease is between 10-30%

According to a specific embodiment, the significant decrease is between 1-30 %.

According to a specific embodiment, the significant decrease is between 5-30%.

According to a specific embodiment, the significant decrease is between 1-50%.

According to a specific embodiment, the significant decrease is between 1-20%.

According to a specific embodiment, the significant decrease is between 1-10%.

According to a specific embodiment, the cell has a 5-hmc prevalence lower than 0.01 % of total DNA bases.

According to a specific embodiment, the cell has a 5-hmc prevalence lower than 0.005 % of total DNA bases.

According to a specific embodiment, the cell has a 5-hmc prevalence lower than 0.004 % of total DNA bases.

According to a specific embodiment, the cell has a 5-hmc prevalence lower than 0.003 % of total DNA bases.

According to a specific embodiment, the cell has a 5-hmc prevalence lower than 0.002 % of total DNA bases.

According to additional or alternative embodiment, the cell has a 5-hmc prevalence lower than 0.0019.

Accordingly, according to a specific embodiment, the cell has a 5-hmc prevalence lower than 0.0018 % of total DNA bases.

According to a specific embodiment, the cell has a 5-hmc prevalence lower than 0.0017 % of total DNA bases.

According to a specific embodiment, the cell has a 5-hmc prevalence lower than 0.0016 % of total DNA bases.

According to a specific embodiment, the cell has a 5-hmc prevalence lower than 0.0015 % of total DNA bases.

According to a specific embodiment, the cell has a 5-hmc prevalence lower than 0.0014 % of total DNA bases.

According to a specific embodiment, the cell has a 5-hmc prevalence lower than 0.0013 % of total DNA bases.

According to a specific embodiment, the cell has a 5-hmc prevalence lower than 0.0012 % of total DNA bases.

According to a specific embodiment, the cell has a 5-hmc prevalence lower than 0.0011 % of total DNA bases.

According to a specific embodiment, the cell has a 5-hmc prevalence lower than
0.0001 % of total DNA bases.

According to a specific embodiment, the cancer is a soft tissue tumor or a solid tumor and the cell is an immune cell e.g., PBMC e.g., a lymphocyte. According to such an embodiment, the assay may be performed by a simple blood test that is non-invasive.

According to a specific embodiment the cancer is a solid tumor and the cell is of the cancer *(in situ* metastasis).

As used herein the term "diagnosing" refers to determining presence or absence of a pathology (e.g., a disease, disorder, condition or syndrome), classifying a pathology or a symptom, determining a severity of the pathology, monitoring pathology progression (i.e., repeating the determination 2 or more times throughout the subject's life time), monitoring treatment (i.e., 5-hmc level following and optionally prior-to treatment e.g., with an anti-cancer drug), forecasting an outcome of a pathology and/or prospects of recovery and screening of a subject for a specific disease.

The sample can be obtained using methods known in the art such as using a syringe with a needle, a scalpel, Eine needle aspiration (FNA), catheter, gastrointestinal endoscopy (e.g., colorectal endoscopy, gastroendoscopy) and the like.

As used herein "DNA" is cellular DNA or cell-free DNA.

Once the sample is obtained, DNA is extracted using methods which are well known in the art, involving tissue mincing, cell lysis, protein extraction and/or DNA precipitation using 2 to 3 volumes of 100% ethanol, rinsing in 70% ethanol, pelleting, drying and resuspension in water or any other suitable buffer (e.g., Tris-EDTA). Preferably, following such procedure, DNA concentration is determined such as by measuring the optical density (OD) of the sample at 260 nm (wherein 1 unit OD=50 μg/ml DNA). To determine the presence of proteins in the DNA solution, the OD 260/OD 280 ratio is determined.

According to some embodiments of the invention, screening of the subject for a specific disease is followed by substantiation of the screen results using gold standard methods (e.g., biopsy, ultrasound, CT, MRI, TAA expression, cytomorphometry, clinical tissue staining (e.g., Vital iodine stain, Tblue stain)).

As used herein "5-hmc prevalence" refers to the percentage of 5-hmc in the entire genome of the cell (i.e., total deoxynucleotides).

Non-limiting examples of cancers which can be diagnosed by the method of this aspect of some embodiments of the invention can be any solid or non-solid cancer and/or cancer metastasis, including, but is not limiting to, tumors of the gastrointestinal tract (colon carcinoma, rectal carcinoma, colorectal carcinoma, colorectal cancer, colorectal adenoma, hereditary nonpolyposis type 1, hereditary nonpolyposis type 2, hereditary

nonpolyposis type 3, hereditary nonpolyposis type 6; colorectal cancer, hereditary nonpolyposis type 7, small and/or large bowel carcinoma, esophageal carcinoma, tylosis with esophageal cancer, stomach carcinoma, pancreatic carcinoma, pancreatic endocrine tumors), endometrial carcinoma, dermatofibrosarcoma protuberans, gallbladder carcinoma, Biliary tract tumors, prostate cancer, prostate adenocarcinoma, renal cancer (e.g., Wilms' tumor type 2 or type 1), liver cancer (e.g., hepatoblastoma, hepatocellular carcinoma, hepatocellular cancer), oral squamous cell carcinoma (OSCC), bladder cancer, embryonal rhabdomyosarcoma, germ cell tumor, trophoblastic tumor, testicular germ cells tumor, immature teratoma of ovary, uterine, epithelial ovarian, sacrococcygeal tumor, choriocarcinoma, placental site trophoblastic tumor, epithelial adult tumor, ovarian carcinoma, serous ovarian cancer, ovarian sex cord tumors, cervical carcinoma, uterine cervix carcinoma, small-cell and non-small cell lung carcinoma, nasopharyngeal, breast carcinoma (e.g., ductal breast cancer, invasive intraductal breast cancer, sporadic; breast cancer, susceptibility to breast cancer, type 4 breast cancer, breast cancer-1, breast cancer-3; breast-ovarian cancer), squamous cell carcinoma (e.g., in head and neck), neurogenic tumor, astrocytoma, ganglioblastoma, neuroblastoma, lymphomas (e.g., Hodgkin's disease, non-Hodgkin's lymphoma, B cell, Burkitt, cutaneous T cell, histiocytic, lymphoblastic, T cell, thymic), gliomas, adenocarcinoma, adrenal tumor, hereditary adrenocortical carcinoma, brain malignancy (tumor), various other carcinomas (e.g., bronchogenic large cell, ductal, Ehrlich-Lettre ascites, epidermoid, large cell, Lewis lung, medullary, mucoepidermoid, oat cell, small cell, spindle cell, spinocellular, transitional cell, undifferentiated, carcinosarcoma, choriocarcinoma, cystadenocarcinoma), ependimoblastoma, epithelioma, erythroleukemia (e.g., Friend, lymphoblast), fibrosarcoma, giant cell tumor, glial tumor, glioblastoma (e.g., multiforme, astrocytoma), glioma hepatoma, heterohybridoma, heteromyeloma, histiocytoma, hybridoma (e.g., B cell), hypernephroma, insulinoma, islet tumor, keratoma, leiomyoblastoma, leiomyosarcoma, leukemia (e.g., acute lymphatic, acute lymphoblastic, acute lymphoblastic pre-B cell, acute lymphoblastic T cell leukemia, acute - megakaryoblastic, monocytic, acute myelogenous, acute myeloid, acute myeloid with eosinophilia, B cell, basophilic, chronic myeloid, chronic, B cell, eosinophilic, Friend, granulocytic or myelocytic, hairy cell, lymphocytic, megakaryoblastic, monocytic, monocytic-macrophage, myeloblastic, myeloid, myelomonocytic, plasma cell, pre-B cell, promyelocytic, subacute, T cell, lymphoid neoplasm, predisposition to myeloid malignancy, acute nonlymphocytic leukemia), lymphosarcoma, melanoma, mammary tumor, mastocytoma, medulloblastoma, mesothelioma, metastatic tumor, monocyte tumor, multiple myeloma, myelodysplastic syndrome, myeloma, nephroblastoma, nervous tissue glial tumor, nervous tissue neuronal tumor, neurinoma, neuroblastoma, oligodendroglioma, osteochondroma, osteomyeloma, osteosarcoma (e.g., Ewing's), papilloma, transitional cell, pheochromocytoma, pituitary tumor (invasive), plasmacytoma, retinoblastoma, rhabdomyosarcoma, sarcoma (e.g., Ewing's, histiocytic cell, Jensen, osteogenic, reticulum cell), schwannoma, subcutaneous tumor, teratocarcinoma (e.g., pluripotent), teratoma, testicular tumor, thymoma and trichoepithelioma, gastric cancer, fibrosarcoma, glioblastoma multiforme; multiple glomus tumors, Li-Fraumeni syndrome, liposarcoma, lynch cancer family syndrome II, male germ cell tumor, mast cell leukemia, medullary thyroid, multiple meningioma, endocrine neoplasia myxosarcoma, paraganglioma, familial nonchromaffin, pilomatricoma, papillary, familial and sporadic, rhabdoid predisposition syndrome, familial, rhabdoid tumors, soft tissue sarcoma, and Turcot syndrome with glioblastoma.

According to a specific embodiment, the cancer is of the gastrointestinal system (GI) e.g., colon cancer or rectal cancer e.g., colorectal cancer or a pre-malignant lesion.

According to a specific embodiment, the cancer is leukemia.

According to a specific embodiment, the cancer is multiple myeloma.

According to a specific embodiment, the cell is selected from tissues which are characterized by particularly low 5-hmc prevalence.

Total DNA bases are the number of bases of DNA measured in the sample.

Measuring total DNA is done using fluorescence intensity measurement of a DNA stain (usually intercalating dye such as YOYO-1, Pico green, Eva green), this measurement is also referred to as (Y). See e.g., for further details describing the correlation between intensity and the number of bases Yuval Ebenstein, Dmitri Torchinsky, Sizing femtogram amounts of dsDNA by single-molecule counting. Nucl. Acids Res., (2016) doi: 10.1093/nar/gkv904.

For example: liver- 0.01%, Heart-0.008%, spleen- 0.005%, Testis- 0.005%, Hela cell line - 0.001%. (Journal of Nucleic Acids Volume 2011, Article ID 870726 and Plos one, December 2010 Volume 5 Issue 12 e15367)

According to the method of this invention, the sample for DNA extraction can be from various tissues or body fluids including but are not limited to tissue biopsy, tissue section, formalin fixed paraffin embedded (FFPE) specimens, blood, plasma, serum, bone marrow, cerebro-spinal fluid, tears, sweat, lymph fluid, saliva, nasal swab or nasal aspirate, sputum, bronchoalveolar lavage, breast aspirate, pleural effusion, peritoneal fluid, glandular fluid, amniotic fluid, cervical swab or vaginal fluid, ejaculate, semen, prostate fluid, urine, conjunctival fluid, duodenal juice, pancreatic juice, bile, and stool.

DNA could be isolated by lysis of cells with lysis buffer containing a sodium salt, tris-HC1, EDTA, and detergents such as sodium dodecyl sulphate (SDS) or cetyltrimethylammonium bromide (CATB). Tissue fragments should be homogenized before lysing. For example, disaggregating of tissue fragments can be performed by stroking 10-50 times, depending an tissue type, with a Dounce homogenizer. DNA can be further purified by mixing with a high concentration of sodium chloride and then adding into a column pre-inserted with a silica gel, a silica membrane, or a silica filter. The DNA that binds to the silica matrix is washed by adding a washing buffer and eluted with TE buffer or water. DNA can also be isolated and purified by using commercially available DNA extraction kits such as QiaAmp tissue kits. Body fluid should be pre-treated under appropriate condition prior to DNA extraction. For example, if a blood sample is used in this invention, anti-coagulants contained in whole blood should be able to inhibit DNAse activity. A suitable anticoagulant may be a chelating agent such as EDTA that prevents both DNAse-caused DNA degradation and clotting of the whole blood samples. If other body fluid samples such as sputum are used, Cells in these kinds of samples can be collected by the procedures described in prior art. For example, collection of cells in a urine sample can simply be achieved by simply centrifugation, while collection of cells in a sputum sample requires DTT treatment of sputum followed by filtering through a nylon gauze mesh filter and then centrifugation. If a stool sample is used, a stool stabilizing and homogenizing reagents should be added to stabilize DNA and remove stool particles. Human DNA fraction from total stool DNA then can be primarily isolated or purified using commercially available stool DNA isolation kits such as Qiagen DNA Stool Mini Kit (using the protocol for human DNA extraction) or be captured by methyl-binding domain (MBD)-based methylated DNA capture methods after total DNA isolation [Zhou H et al., Clinical Chemistry, 2007].

According to a specific embodiment, the DNA molecules are cell-free DNA molecules.

Thus, according to an aspect of some embodiments of the present invention there is provided a method of labeling the epigenetic modification 5-hydroxymethylcytosine (5hmC) along a DNA molecule.

As used herein "5-Methylcytosine" or "5mC" is a methylated form of the DNA base cytosine. When cytosine is methylated, the DNA maintains the same sequence, but the expression of methylated genes can be altered (the study of this is part of the field of epigenetics). 5-Methylcytosine is incorporated in the nucleoside 5-methylcytidine. As used herein "5-Hydroxymethylcytosine" or "5hmC" is a DNA pyrimidine nitrogen base. It is formed from the DNA base cytosine by adding a methyl group and then a hydroxyl group.

As used herein the term "DNA" refers to single stranded DNA or a double stranded DNA which is isolated. The DNA can be a eukaryotic DNA (e.g., rodent or primate e.g., human) in which 5hmC modifications typically occur or a synthetic DNA in which 5hmC modifications may be artificially added.

According to an embodiment of the invention, the DNA molecule is a complementary polynucleotide sequence (cDNA) to which 5hmC modifications have been artificially added, a genomic polynucleotide sequence and/or a composite polynucleotide sequences (e.g., a combination of the above).

As used herein the phrase "complementary polynucleotide sequence" refers to a sequence, which results from reverse transcription of messenger RNA using a reverse transcriptase or any other RNA dependent DNA polymerase. Such a sequence can be subsequently amplified in vivo or in vitro using a DNA dependent DNA polymerase. As used herein the phrase "genomic polynucleotide sequence" refers to a sequence derived (isolated) from a chromosome and thus it represents a contiguous portion of a chromosome.

As used herein the phrase "composite polynucleotide sequence" refers to a sequence, which is at least partially complementary and at least partially genomic. A composite sequence can include some exonal sequences required to encode the polypeptide of the present invention, as well as some intronic sequences interposing therebetween. The intronic sequences can be of any source, including of other genes, and typically will include conserved splicing signal sequences. Such intronic sequences may further include cis acting expression regulatory elements. The length of the DNA molecule may vary. Exemplary ranges include, but are not limited to 1-1 5,000 Kb, reflecting at the high range the size of a human chromosomes (or chromatin). According to some embodiments of the invention, the DNA molecule is longer than 20 Kb. According to some embodiments of the invention, the DNA molecule is longer than 30 Kb. According to some embodiments of the invention, the DNA molecule is longer than 40 Kb. Detection of the labeled DNA molecule can be done at the single molecule level using optical imaging as further described hereinbelow. Alternatively, detection of labeled DNA molecules can be done at the global level, analyzing the presence or level of 5hmC modification of a plurality of DNA molecules at the cell, tissue and organism level, as further described hereinbelow. As mentioned, the methods described herein rely on subjecting the labeled DNA molecule to an imaging method suitable for detecting the labeling agent. Such imaging methods are described herein below and typically do not rely on mass-spectrometry, radioactive assays, or immune assays (e.g., ELISA, DOT-BLOT, immunoprecipitation).

Accordingly, and according to a specific embodiment a fluorescent dye is directly attached to the DNA (not mediated by antibody binding) and the fluorescent signal is detected and quantified.

Thus, according to an embodiment of the invention there is provided a method of labeling the epigenetic modification 5-hydroxymethyl-cytosine (5hmC) along a (single) DNA molecule, the method comprising:

(a) attaching to the DNA molecule a 5hmc specific labeling agent; and

(b) extending the DNA molecule.

Large scale settings may employ imaging without the step of DNA extension b quantifying the ratio between overall DNA signal and overall 5hmC signal (which results in the 5-hmc prevalence).

As mentioned hereinabove and further described hereinbelow, attachment of a 5hmc specific labeling agent to the DNA molecule is effected when analysis is performed in the single molecule level or when a plurality of DNA molecules (global 5hmC analysis) are analyzed.

As used herein "a 5hmC specific labeling agent" refers to a labeling agent that differentiates between 5hmC modification and non-modified cytosine or methylated cytosine (5mC), as described hereinabove. A 5hmC specific labeling agent labels selectively the position or positions where 5hmC modification is present in a DNA molecule, and does not label those positions in a DNA molecule where 5mC or any other epigenetic modification is present. The 5hmC labeling agent according to some embodiments of the present invention is fluorescently detectable. A list of suitable labeling agents is provided hereinafter.

According to some embodiments of the invention, a 5hmC specific labeling agent labels at least 50 %, or at least 70 %, or at least 80 %, or at least 90 % of the a 5hmC modifications in a DNA molecule, including any intermediate within 50-100%.

According to some embodiments of the present invention, a 5hmC specific labeling agent is attached (e.g., covalently) selectively to 5hmC.

In some embodiments, selectively attaching a 5hmC specific labeling agent is effected by: reacting a labeling agent derivatized by a reactive group (herein referred to as a second reactive group) with a DNA molecule in which the 5-hydroxymethylcytosine bases are glycosylated by a glucose molecule derivatized by another reactive group (herein referred to as a first reactive group).

The first and second reactive groups are selected as being chemical compatible to one another. By "chemically compatible" it is meant that the first and second reactive groups

can react with one another so as to form a chemical bond. As used herein, the phrase "reactive group" describes a chemical group that is capable of undergoing a chemical reaction that typically leads to a bond formation. The bond can involve one or more of a covalent bond, an electrostatic bond, a hydrogen bond, aromatic interactions, and any combination thereof.

The bond, according to some embodiments of the present invention, is a covalent bond. Chemical reactions that lead to a bond formation include, for example, cycloaddition reactions (such as the Diels-Alder's reaction, the 1 ,3-dipolar cycloaddition Huisgen reaction, and the similar "dück reaction"), condensations, nucleophilic and electrophilic addition reactions, nucleophilic and electrophilic substitutions, addition and elimination reactions, alkylation reactions, rearrangement reactions and any other known organic reactions that involve a reactive group.

Representative examples of reactive groups include, without limitation, acyl halide, aldehyde, alkoxy, alkyne, amide, amine, aryloxy, azide, aziridine, azo, carbamate, carbonyl, carboxyl, carboxylate, cyano, diene, dienophile, epoxy, guanidine, guanyl, halide, hydrazide, hydrazine, hydroxy, hydroxylamine, imino, isocyanate, nitro, phosphate, phosphonate, sulfinyl, sulfonamide, sulfonate, thioalkoxy, thioaryloxy, thiocarbamate, thiocarbonyl, thiohydroxy, thiourea and urea, as these terms are defined hereinafter.

Exemplary first and second reactive groups that are chemically compatible with one another as described herein include, but are not limited to, hydroxy and carboxylic acid, which form an ester bond; thiol and carboxylic acid, which form a thioester bond; amine and carboxylic acid, which form an amide bond; aldehyde and amine, hydrazine, hydrazide, hydroxylamine, phenylhydrazine, semicarbazide or thiosemicarbazide, which form a Schiff base (imine bond); alkene and diene, which react therebetween via cycloaddition reactions; and reactive groups that can participate in a Click reaction.

Additional examples of pairs of reactive groups (first and second reactive groups) capable of reacting with one another include an azide and an alkyne, an unsaturated carbon-carbon bond (e.g., acrylate, methacrylate, maleimide) and a thiol, an unsaturated carbon-carbon bond and an amine, a carboxylic acid and an amine, a hydroxyl and an isocyanate, a carboxylic acid and an isocyanate, an amine and an isocyanate, a thiol and an isocyanate. Additional examples include an amine, a hydroxyl, a thiol or a carboxylic acid along with a nucleophilic leaving group (e.g., hydroxysuccinimide, a halogen).

It is to be appreciated that for each pair of reactive groups described hereinabove, either reactive group can correspond to the "first reactive group" or to the "second reactive group".

In some embodiments, the first and/or the second reactive groups can be latent groups, which are exposed during the chemical reaction, such that the reacting (e.g., covalent bond formation) is effected once a latent group is exposed. Exemplary such groups include, but are not limited to, reactive groups as described hereinabove, which are protected with a protecting group that is labile under selected reaction conditions.

Examples of labile protecting groups include, for example, carboxylate esters, which may hydrolyzed to form an alcohol and a carboxylic acid by exposure to acidic or basic conditions; silyl ethers such as trialkyl silyl ethers, which can be hydrolysed to an alcohol by acid or fluoride ion; p-methoxybenzyl ethers, which may be hydrolysed to an alcohol, for example, by oxidizing conditions or acidic conditions; t-butyloxycarbonyl and 9-fluorenylmethyloxycarbonyl, which may be hydrolysed to an amine by a exposure to basic conditions; sulfonamides, which may be hydrolysed to a sulfonate and amine by exposure to a suitable reagent such as samarium iodide or tributyltin hydride; acetals and ketals, which may be hydrolysed to form an aldehyde or ketone, respectively, along with an alcohol or diol, by exposure o acidic conditions; acylals (i.e., wherein a carbon atom is attached to two carboxylate groups), which may be hydrolysed to an aldehyde of ketone, for example, by exposure to a Lewis acid; orthoesters (i.e., wherein a carbon atom is attached to three alkoxy or aryloxy groups), which may be hydrolysed to a carboxylate ester (which may be further hydrolysed as described hereinabove) by exposure to mildly acidic conditions; 2-cyanoethyl phosphates, which may be converted to a phosphate by exposure to mildly basic conditions; methylphosphates, which may be hydrolysed to phosphates by exposure to strong nucleophiles; phosphates, which may be hydrolysed to alcohols, for example, by exposure to phosphatases; and aldehydes, which may be converted to carboxylic acids, for example, by exposure to an oxidizing agent.

According to some embodiments of the present invention, a linking moiety is formed as a result of a bond-forming reaction between two (first and second) reactive groups.

Exemplary linking moieties, according to some embodiments of the present invention, which are formed between a first and a second reactive groups as described herein include without limitation, amide, lactone, lactam, carboxylate (ester), cycloalkene (e.g., cyclohexene), heteroalicyclic, heteroaryl, triazine, triazole, disulfide, imine, aldimine, ketimine, hydrazone, semicarbazone and the likes. Other linking moieties are defined hereinbelow.

For example, a reaction between a diene reactive group and a dienophile reactive group, e.g. a Diels-Alder reaction, would form a cycloalkene linking moiety, and in most cases a cyclohexene linking moiety. In another example, an amine reactive group would form an amide linking moiety when reacted with a carboxyl reactive group. In another example, a hydroxyl reactive group would form an ester linking moiety when reacted with a carboxyl reactive group. In another example, a sulfhydryl reactive group would form a disulfide (-S-S-) linking moiety when reacted with another sulfhydryl reactive group under oxidation conditions, or a thioether (thioalkoxy) linking moiety when reacted with a halo reactive group or another leaving-reactive group. In another example, an alkynyl reactive group would form a triazole linking moiety by "dick reaction" when reacted with an azide reactive group.

The "dick reaction", also known as "dick chemistry" is a name often used to describe a stepwise variant of the Huisgen 1,3-dipolar cycloaddition of azides and alkynes to yield 1,2,3-triazole. This reaction is carried out under ambient conditions, or under mild microwave irradiation, typically in the presence of a Cu(I) catalyst, and with exclusive regioselectivity for the 1 ,4-disubstituted triazole product when mediated by catalytic amounts of Cu(I) salts [V. Rostovtsev, L. G. Green, V. V. Fokin, K. B. Sharpless, Angew. Chem. Int. Ed. 2002, 41, 2596; H.C. Kolb, M. Finn, K.B. Sharpless, Angew Chem., Int. Ed. 2001, 40, 2004].

As demonstrated in the Examples section that follows, the "dick reaction" is particularly suitable in the context of embodiments of the present invention since it can be carried out under conditions which are non-distructive to DNA molecules, and it affords attachment of a lebeling agent to 5hmC in a DNA molecule at high chemical yields using mild conditions in aqueous media. The selectivity of this reaction allows to perform the reaction with minimized or nullified use of protecting groups, which use often results in multistep cumbersome synthetic processes.

In exemplary embodiments, the first and second reactive groups comprise (in no particular order) an azide and an alkyne. These two reactive groups may combine to form a triazole ring, as defined herein, as a linking moiety. These two reactive groups thus combine to attach a labeling agent to the 5hmC in the DNA molecule by a mechanism referred to as "dick" chemistry, as defined herein. The term "derivatized", as used herein in the context of a labeling agent and a glucose, means that the labeling agent and/or the glucose are substituted, or are modified by substituting a position thereof, by a chemical moiety that comprises the respective (first or second) reactive group.

For example, a labeling agent derivatized by a second reactive group, as described herein, means that a labeling agent as described herein is modified so as to comprise a second reactive group as described herein, by substituting a position thereof with a chemical moiety that comprises the second reactive group. Alternatively, the second reactive group or a chemical moiety comprising the second reactive group already forms a part in a labeling agent as a substituent. A chemical moiety that comprises the second reactive group can be the second reactive group *per se* or, for example, a spacer moiety that includes, and preferably terminates with, the second reactive group.

As used herein, the phrase "spacer moiety" describes a chemical moiety that typically extends between two chemical moieties and is attached to each of the chemical moieties via covalent bonds. The spacer moiety may be linear or cyclic, be branched or unbranched, rigid or flexible.

According to some embodiments of the present invention, the spacer moieties are selected such that they allow and/or promote the one or both of attachment of a second reactive group to the labeling agent and attachment of the labeling agent to the 5hmC in a DNA molecule. Such traits can be selected for in terms of spacer's length, flexibility, structure and specific chemical reactivity or lack thereof.

Exemplary spacer moieties include, but are not

limited to, alkyl, alkenyl, alkynyl, cycloalkyl, heteroalicyclic, aryl, heteroaryl and/or a hydrocarbon chain having 1-20 carbon atoms and ending or interrupted by at least one heteroatom selected from the group consisting of O, S and N and/or containing from 0 to 19 unsaturated carbon-carbon or carbon-heteroatom bonds. Additional spacer moieties include, without limitation, -CH2-, $-CH_2-O-$, -(CH2)2-, - $(CH_2)_2-O-$, -(CH2)3-, -(CH2)3-O-, -(CH2)4-, -(CH2)5-, -(CH2)6, -(CH(CH3))-CH2-, -CH=CH-CH=CH-, $-CH_2CH(OH)CH_2-$, $CH_2-O-CH_2-O-$, - $(CH_2)_2-O-(CH_2)_2-$, $-(CH_2)_2-O-(CH_2)_2-O-$, - $CH_2-mC_6H_4-CH_2-$, $-CH_2-mC_6H_4-CH_2-O-$, - $CH_2-pCeH_4-CH_2-$, -CH2-pC6H4-CH2-O-, $-CH_2-NHC0-$,$-C_6H_4-NHC0-$,$-CH_2-O-CH_2-$ and -CH=CH-CH2-NH-(CH2)2-, and any combination thereof. Short polymeric chains, such as, for example, polyalkylene glycols, are also contemplated. In exemplary embodiments, a second reactive group as described herein is attached to a labeling agent via a spacer moiety, while exploiting functional groups present in the labeling agent for attaching thereto the spacer moiety which terminates with the second reactive group.A labeling agent derivatized by a second reactive group as described herein can be selected and prepared using conventional chemical reactions, or can be a commercially available derivatized labeling agent. In exemplary embodiments, the second reactive group is an alkyne and the labeling agent is derivatized by a chemical moiety that comprises an alkyne, as described herein. Such a chemical moiety can comprise, for example, benzocyclooctyne (DIBO), and can be attached to the labeling agent via a spacer as described herein. According to some of these embodiments, the second reactive group is a "strained alkyne". A "strained alkyne" is a cycloalkyne, preferably substituted by one or more groups that render it highly strained, for example, cyclopropyls, benzyls, and others. Examples of known strained alkynes include, but are not limited to, the following:

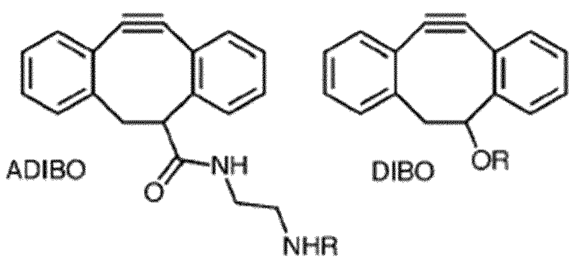

ADIBO DIBO

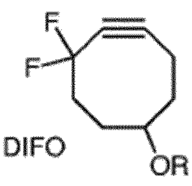

DIFO

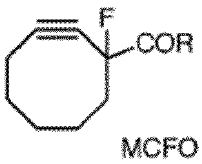

MCFO

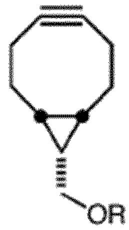

BCN

The use of a strained alkyl allows performing the dick reaction without using a copper catalyst. A glucose derivatized by a first reactive group describes a glucose moiety that is substituted at one position thereof by a chemical moiety that comprises the first reactive group, as described herein. For example, one of the hydroxy groups of a glucose can be substituted by a chemical moiety that comprises the first reactive group or can be used to attach to the glucose the chemical moiety that comprises the first reactive group, via chemical reactions that involve a hydroxy group, as described herein. A chemical moiety that comprises the first reactive group can be the first reactive group per se or, for example, a spacer moiety, as described herein, that includes, or terminates with, the first reactive group. In exemplary embodiments, one of the hydroxy groups of a glucose is substituted (replaced) by a chemical moiety that comprises the first reactive group.

Chemical reactions for substituting a hydroxy group are well known in the art. In some of these embodiments, the first reactive group is azide and a hydroxy at position 6 of the glucose is substituted by an azide group. An exemplary synthetic pathway for preparing 6-azido-glucose is depicted in Figure 1 of WO2014/191981.

According to some embodiments of the invention, a DNA molecule in which the 5-hydroxymethylcytosine bases are glycosylated by a glucose molecule derivatized by the first reactive group is prepared, while utilizing a glucose derivatized by the first reactive group, as described herein.

In some embodiments, a selective introduction of a glucose derivatized by the first reactive group to 5-hydroxymethylcytosines in a DNA molecule comprises incubating the DNA molecule with f3-glucosyltransferase and a uridine diphosphoglucose (UDP-Glu) derivatized by the first reactive group.

A DNA beta-glucosyltransferase (EC 2.4.1.27) is an enzyme that catalyzes the chemical reaction in which a beta-D-glucosyl residue is transferred from UDP-glucose to an hydroxymethylcytosine residue in DNA. This enzyme belongs to the family of glycosyltransferases, specifically the hexosyltransferases. The systematic name of this enzyme class is UDP-glucose:DNA beta-D-glucosyltransferase. Other names in common use include T4-HMC-beta- glucosyl transferase, T4-beta-glucosyl transferase, phage beta-glucosyltransferase, UDP glucose-DNA beta glucosyltransferase, and uridine diphosphoglucose-deoxyribonucleate beta-glucosyltransferase. In certain aspects, the a I3-glucosyltransferase is a His-tag fusion protein.

In other embodiments, the protein may be used without the His-tag (hexa-histidine tag shown above) portion. A uridine diphosphoglucose (UDP-Glu) derivatized by the first reactive group is meant to describe a uridine diphosphoglucose in which the glucose moiety is derivatized by a first reactive group, according to any one of the embodiments described herein. In some embodiments, the uridine diphosphoglucose (UDP-G1u) derivatized by the first reactive group is a UDP-6-$N_3$-Glucose. A UDP-6-$N_3$-Glucose, or any other uridine diphosphoglucose (UDP-Glu) derivatized by the first reactive group, can be prepared by chemical synthesis, while utilizing, for example, a 6-azido glucose or any other derivatized glucose, or can be a commercially available product.

In some embodiments, the UDP-6-$N_3$-Glucose, or any other uridine diphosphoglucose (UDP-Glu) derivatized by the first reactive group, is prepared by enzymatically-catalyzed reactions, as exemplified in further detail hereinafter. Once a glucose derivatized by a first reactive group is introduced to 5-hmCs in a DNA molecule, the DNA molecule is reacted with a labeling agent derivatized by a compatible second reactive group, as described herein.

As discussed hereinabove, in some embodiments, the reaction involves a dick chemistry reaction. According to some embodiments of the invention, the dick chemistry reaction is free of a copper catalyst, namely, is effected without the presence of a copper catalyst or an other catalyst that may adversely affect the DNA molecule. For any one of the embodiments described herein throughout, the phrase "labeling agent" refers to a detectable moiety or a probe. Exemplary labeling agents which are suitable for use in the context of these embodiments include, but are not limited to, a fluorescent agent, a radioactive agent, a magnetic agent, a chromophore, a bioluminescent agent, a chemiluminescent agent, a phosphorescent agent and a heavy metal cluster, as well as any other known detectable agents.

In some embodiments, the labeling agent is an agent that is detectable by spectrophotometric measurements, and/or which can be utilized to produce optical imaging. Such agents include, for example, chromophores, fluorescent agents, phosphorescent agents, and heavy metal clusters.

As used herein, the term "chromophore" refers to a chemical moiety that, when attached to another molecule, renders the latter colored and thus visible when various spectrophotometric measurements are applied.

The phrase "fluorescent agent" refers to a compound that emits light at a specific wavelength during exposure to radiation from an external source.

The phrase "phosphorescent agent" refers to a compound emitting light without appreciable heat or external excitation as by slow oxidation of phosphorous.

A heavy metal cluster can be for example a cluster of gold atoms used, for example, for labeling in electron microscopy techniques (e.g., AFM).

The term "bioluminescent agent" describes a substance which emits light by biochemical process.

The term "chemiluminescent agent" describes a substance which emits light as the result of a chemical reaction.

According to some embodiments of the invention, the labeling agent is a fluorescent labeling agent.

A fluorescent agent can be a protein, quantum dots or small molecules.

Common dye families include, but are not limited to Xanthene derivatives: fluorescein, rhodamine, Oregon green, eosin, Texas red etc.; Cyanine derivatives: cyanine, indocarbocyanine, oxacarbocyanine, thiacarbocyanine and merocyanine; Naphthalene derivatives (dansyl and prodan derivatives); Coumarin derivatives; oxadiazole derivatives: pyridyloxazole, nitrobenzoxadiazole and benzoxadiazole; Pyrene derivatives: cascade blue etc.; BODIPY (Invitrogen); Oxazine derivatives: Nile red, Nile blue, cresyl violet, oxazine 170 etc.; Acridine derivatives: proflavin, acridine orange, acridine yellow etc.; Arylmethine derivatives: auramine, crystal violet, malachite green; CF dye (Biotium); Alexa Fluor (Invitrogen); Atto and Tracy (Sigma Aldrich); FluoProbes (Interchim); Tetrapyrrole derivatives: porphin, phtalocyanine, bilirubin; cascade yellow; azure B; acridine orange; DAPI; Hoechst 33258; lucifer yellow; piroxicam; quinine and anthraqinone; squarylium; oligophenylenes; and the like. Other fluorophores include: Hydroxycoumarin; Aminocoumarin; Methoxycoumarin; Cascade Blue; Pacific Blue; Pacific Orange; Lucifer yellow; NBD; R-Phycoerythrin (PE); PE-Cy5 conjugates; PE-Cy7 conjugates; Red 613; PerCP; TruRed; FluorX; Fluorescein; BODIPY-FL; TRITC; X-Rhodamine; Lissamine Rhodamine B; Texas Red; Aliaphycocyanin; APC-Cy7 conjugates.

Alexa Fluor dyes (Molecular Probes) include: Alexa Fluor 350, Alexa Fluor 405, Alexa Fluor 430, Alexa Fluor 488, Alexa Fluor 500, Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 647, Alexa Fluor 660, Alexa Fluor 680, Alexa Fluor 700, Alexa Fluor 750, and Alexa Fluor 790.

Cy Dyes (GE Healthcare) include Cyt, Cy3, Cy3B, Cy3.5, Cy5, Cy5.5 and Cy7.

Nucleic acid probes include Hoechst 33342, DAPI, Hoechst 33258, SYTOX Blue, ChromomycinA3, Mithramycin, YOY0-1, Ethidium Bromide, Acridine Orange, SYTOX Green, TOTO-1, TO-PRO-1, TO-PRO: Cyanine Monomer, Thiazole Orange, Propidium Iodide (PI), LDS 751, 7-AAD, SYTOX Orange, TOTO-3, TO-PRO-3, and DRAQ5.

Cell function probes include Indo-1, Fluo-3, DCFH, DHR, SNARF.

Fluorescent proteins include Y66H, Y66F, EBFP, EBFP2, Azurite, GFPuv, T- Sapphire, Cerulean, mCFP, ECFP, CyPet, Y66W, mKeima-Red, TagCFP,

AmCyanl, mTFP1, S65A, Midoriishi Cyan, Wild Type GFP, S65C, TurboGFP, TagGFP, S65L, Emerald, S65T (Invitrogen), EGFP (Ciontech), Azami Green (MBL), ZsGreenl (Clontech), TagYFP (Evrogen), EYFP (Clontech), Topaz, Venus, mCitrine, YPet, Turbo YFP, ZsYellow 1 (Clontech), Kusabira Orange (MBL), mOrange, mKO, TurboRFP (Evrogen), tdTomato, TagRFP (Evrogen), DsRed (Clontech), DsRed2 (Clontech), mStrawberry, TurboFP602 (Evrogen), AsRed2 (Clontech), mRFP1, J-Red, mCherry, HcRedl (Clontech), Katusha, Kate (Evrogen), TurboFP635 (Evrogen), mP!um, and mRaspberry.

It is to be noted that, in some embodiments, each of the labeling agents (e.g., fluorophores) is attached to the DNA molecule by means of dick chemistry and that the reagents used for the reaction are derivatives of the labeling agent, which include a reactive group as described herein.

Exemplary fluorescent agents include, but are not limited to, Alexa fluor dyes, Cy Dyes, Atto dyes, TAMRA dyes, etc., such as, for example, described in the Examples section that follows.

According to some embodiments of the invention, analyzing 5hmC content is done without subjecting the DNA molecule to fragmentation.

As mentioned, the DNA molecule is immobilized on a solid phase.

According to some embodiments of the invention, the extending is linearly extending.

According to some embodiments of the invention, the extending is effected by depositing the DNA molecule on a surface or extending the DNA molecule in a nanochannel. As used herein "extended DNA molecule" or "elongated DNA molecule" whichis interchangeably used herein refers to a single or plurality elongated and fixed (i.e., immobilized) DNA.

According to some embodiments of the invention, the extended DNA molecules are elongated and fixed in a controllable manner directly onto a solid, planar surface. According to a specific embodiment, this solid, planar surface contains a positive charge density which has been controllably modified such that the single nucleic acid molecules will exhibit an optimal balance between the critical parameters of nucleic acid elongation state, degree of relaxation stability and biological activity. Further, methods, compositions and assays are described by which such an optimal balance can precisely and reproducibly be achieved.

According to alternative or additional embodiments, the single nucleic acid molecules are elongated via flow-based techniques. In such an embodiment, a single nucleic acid molecule is elongated, manipulated (via, for example, a regio-specific restriction digestion), and/or analyzed in a laminar flow elongation device. Such a laminar flow elongation devices and methods of elongating or extending DNA are described in U.S. Patent Application 20030124611.

The elongated, individual labeled DNA molecules can then be utilized in a variety of ways which have applications for the analysis of nucleic acid at the genome level. For example, such nucleic acid molecules may be used to generate ordered, high resolution single nucleic acid molecule restriction maps. This method is referred to herein as "optical mapping" or "optical restriction mapping". Additionally, methods are presented whereby specific nucleotide sequences present within the elongated nucleic acid molecules can be identified. Such methods are referred to herein as "optical sequencing". The optical mapping and optical sequencing techniques can be used independently or in combination on the same individual nucleic acid molecules. Additionally, methods are also presented for the imaging and sizing of the elongated single nucleic acid molecules. These imaging techniques may, for example, include the use of fluorochromes, microscopy and/or image processing computer software and hardware.

Further description of DNA extension is provided hereinbelow and in the Examples section which follows.

According to some embodiments of the invention, step (b, extending) is effected following step (a, attaching to the DNA molecule a 5hmc specific labeling agent). However, it will be appreciated that extending the DNA molecule can be done prior to step (a). According to the invention, the method further comprises attaching to the DNA molecule an additional labeling agent distinct of the 5hmc specific labeling agent. According to some embodiments of the invention, the additional labeling agent is an epigenetic modification specific labeling agent. Examples of such modifications include but are not limited to 5-methylcytosine (5mC), histone acetylation and the like.

According to some embodiments of the invention, the additional labeling agent is a non-epigenetic modification specific labeling agent. Examples of such stains and dyes include DNA fluorescent dyes such as cyanine nucleic acid stains, which are essentially non-fluorescent in the absence of nucleic acids and exhibit significant fluorescence enhancements upon DNA binding. The stain may be cell permeant or impermeant.

Such stains are available from Molecular Probes (e.g., YOYO-1. TOTO, S YT 0 X, POPO-1, BOB 0-1, LOLO- 1, J OJ 0-1 etc.). Alternatively, non-fluorescent stains can be used as further described hereinbelow.

Still further, high throughput methods for utilizing such single nucleic acid molecules in genome analysis are presented. In one embodiment of such high throughput methods, rapid optical mapping approaches are described for the creation of high-resolution restriction maps. In such an embodiment, single nucleic acid molecules are elongated, fixed and gridded to high density onto a solid surface. These molecules can then be digested with appropriate restriction enzymes for the map construction. In an alternative embodiment, the single nucleic acid molecules can be elongated, fixed

and gridded at high density onto a solid surface and utilized in a variety of optical sequencing-based diagnostic methods. In addition to speed, such diagnostic grids can be reused. Further, the high throughput and methods can be utilized to rapidly generate information derived from procedures which combine optical mapping and optical sequencing methods.

According to an aspect of some embodiments of the present invention there is provided a method of *in-situ* imaging a DNA molecule, the method comprising:

(a) attaching a labeling agent to the DNA molecule as described herein; and
(b) subjecting the DNA molecule to an imaging method suitable for detecting the labeling agent.Other labeling agents, as described herein, are also contemplated and respective imaging methods are utilized accordingly.

According to some embodiments of the invention, the method further comprises generating an optical image of the DNA molecule following the imaging.

According to an aspect of some embodiments of the present invention, there is provided an extended DNA molecule comprising at least one 5hmc-specific labeling agent. According to an aspect of some embodiments of the present invention, there is provided a DNA molecule comprising at least two different labeling agents, wherein a first labeling agent of the at least two different labels is a 5hmc-specific labeling agent. According to some embodiments of the invention, the 5hmc-specific labeling agent is attached to the DNA molecule by reacting a labeling agent derivatized by a second reactive group with a DNA molecule in which the 5-hydroxymethylcytosines are glycosylated by a glucose molecule derivatized by a first reactive group, wherein the first and second reactive groups are chemically compatible to one another, as described in any one of the embodiments pertaining to attaching a 5hmc-specific labeling agent to a DNA molecule of the present invention. According to some embodiments of the invention, one the first and second reactive groups is azide and the other is alkyne, such that attaching the labeling agent to the DNA molecule is effected by a dick chemistry, as described herein.

According to some embodiments of the invention, a second labeling agent of the at least two different labeling agents is a 5mc-specific labeling agent.

According to some embodiments of the invention, a second labeling agent of the at least two different labeling agents is for an epigenetic modification.

According to some embodiments of the invention, a second labeling agent of the at least two different labeling agents is for a non-epigenetically modified base.

As used herein "distinct" or "different" labels refer to labels which can be distinguished upon visualization. Thus, in fluorescence labeling one label may be red fluorescence while the other can be blue fluorescence.

According to some embodiments of the invention, the DNA molecule is extended.

According to an aspect of some embodiments of the present invention, there is provided a composition-of-matter comprising the DNA molecule.

According to some embodiments of the invention, the DNA molecule is surface deposited or extended in a microchannel.

The present invention also envisages detecting 5hmC in non-immobilized biological samples.

Thus, according to an aspect of some embodiments of the present invention there is provided a method of detecting 5-hydroxymethyl-cytosine (5hmC) in a DNA sample the method comprising:

(a) reacting the DNA sample with a 5hmc-specific fluorescent agent under conditions which allow staining of the DNA sample with said 5hmc-specific labeling agent so as to obtain a 5hmC-labeled DNA sample; and
(b) measuring fluorescence intensity of said 5hmC-labeled DNA sample (X)

and DNA stain intensity (Y), wherein a ratio between X to Y is indicative of presence or level of 5hmC in the DNA sample.

As used herein the term "fluorescence intensity" refers to the intensity of the fluorescent probe.

It will be appreciated that for intercalation based staining of DNA, changes in probe concentration, by dilution of the sample, for example, can, influence the fluorescence intensity of the DNA due to the change in equilibrium. For this reason, DNA preparations are typically not washed to remove unbound probe; otherwise the equilibrium will be interrupted. It will be appreciated that the unbound probe typically does not fluoresce and, hence, demonstrates low background fluorescence. The intensity is measured at an excitation and emission values which depend on the probe.

As used herein "absorbance" refers to DNA light absorbance at 260 nm which is a measure for DNA quantity. At this wavelength, DNA typically exhibits absorbance maxima. As used herein "DNA stain intensity" refers to a non-specific DNA stain that labels the bases globally thus giving a measure of total nucleotides in the sample (such DNA labels are described hereinabove).

According to a specific embodiment, the ratio is compared to a ratiometric calibration curve.

The calibration curve can be generated by using DNA samples of known percentage of 5hmC labeled using the same methodology as the test DNA sample.

The methodology described herein, according to some embodiments of the present invention can be used to detect global 5hmC modification.

As used herein "global 5hmC modification" refers to the detection of 5hmC of a plurality of DNA molecules which are in a non-immobilized state. The sample may be a heterogeneous sample.

According to a specific embodiment, this methodology is more sensitive than adsorption measurement of labeled 5hmC. Thus, as shown in Example 1 of the Examples section which follows, measuring the ratio between the fluorescence signal of labeled 5hmC and the absorption of DNA at 260 nm allowed to detect down to 0.004 % 5hmC/dN from a sample extracted from liver, with a sample concentration of 136 ng/μ.1

in 20 μ1 volume and 0.02 % 5hmC/dN from a DNA sample concentration of only 82 ng/μl in 20 μ1 volume (1.6 μg).

It is contemplated that the threshold of sensitivity or the limit of detection is about 0.0022 % 5hmC/dN.

The concentration of the DNA in the test sample depends an the level (e.g., %) of hmC in the tissue. Thus, a higher DNA concentration is required for tissues with lower levels hmC.In general when assayed using a plate reader, the concentration of DNA that can be read is up to 350 ng/μ1 DNA without having signal saturation (e.g., 1350 ng/μ1). This concentration of DNA is high enough for detection % hmC at low-% hmC-containing tissues such as spleen and liver. However for tissues containing even lower %hmC, concentrated DNA samples (e.g., 100 ng/μ1 to 100 μg/μ1) may be measured for their fluorescence intensity and then diluted for measuring their DNA concentration. 1 pg-/μ1-100 μg/μ1, lpg/p1 -50 μg/μ1, e.g., 5ng/p1-5p g/μ1.

According to a specific embodiment, the volume of the sample is between 1-50 μ1 or 10-20 ul for the detection of hmC in genomic DNA in multi well plate.

Alternatively, the sample can be subjected to optical imaging by extending the molecules on slides (immobilizing the DNA molecules) as described herein. The amount of DNA is measured by length or fluorescence intensity of the intercalating DNA stain, and the amount of 5-hmC is determined by counting the fluorescent spots generated along the DNA by the labeled 5-hmC or measuring their intensity. The position of the modification can also be analyzed using enzymes which are sensitive to

bulky residues i.e., the modification of the 5hmC with $N_3$-5-gmC. Presence of $N_3$-5-g group on the DNA template strand will interfere with the synthesis of a nucleic acid strand by DNA polymerase or RNA polymerase, or the efficient cleavage of DNA by a restriction endonuclease (e.g., Msp 1) or inhibition of other enzymatic modifications of nucleic acid containing 5-hmC. As a result, primer extensions or other assays can be employed, for example, to evaluate a partially extended primer of certain length and the modification sites can be revealed by sequencing the partially extended primers.

The sensitivity of the method may be even augmented by selecting a specific cell type (e.g., lymphocyte from PBMCs) such as by using cell sorting (e.g., FACS, magenic beads etc.).

It will be appreciated that the DNA may be stretched through nanochannels for quantifying the 5hmC. Figure 4 shows DNA extracted from human PBMCs and stretched in nanochannel arrays (BioNano Genomics): DNA molecules in blue, red dots are genetic tags for mapping to the genome and green dots are 5hmC.

As used herein the term "about" refers to ± 10 %.

The terms "comprises", "comprising", "includes", "including", "having" and their conjugates mean "including but not limited to". The term "consisting of' means "including and limited to". The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation an the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range. Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

When reference is made to particular sequence listings, such reference is to be understood to also encompass sequences that substantially correspond to its complementary sequence as including minor sequence variations, resulting from, e.g., sequencing errors, cloning errors, or other alterations resulting in base substitution, base deletion or base addition, provided that the frequency of such variations is less than 1 in 50 nucleotides, alternatively, less than 1 in 100 nucleotides, alternatively, less than 1 in 200 nucleotides, alternatively, less than 1 in 500 nucleotides, alternatively, less than 1 in 1000 nucleotides, alternatively, less than 1 in 5,000 nucleotides, alternatively, less than 1 in 10,000 nucleotides.

It is appreciated that certain features of the invention, which are, for clarity,

described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements. Various embodiments and aspects of the present invention as delineated hereinabove and as claimed in the claims section below find experimental support in the following examples.

## EXAMPLES

Reference is now made to the following examples, which together with the above descriptions, illustrate the invention in a non limiting fashion. Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumesl-lll Cellis, J. E., ed. (1994); "Current Protocols in Immunology" Volumes I-III Coligan J.

E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for 1example, U.S. Pat. Nos. 3,791,932; 3,839,153; 3,850,752; 3,850,578; 3,853,987;

3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984);
"Nucleic Acid Hybridization" Hames, B. D., and Higgins S. J., eds. (1985); "Transcription and Translation" Hames, B. D., and Higgins S. J., eds. (1984);
"Animal Cell Culture" Freshney, R. I., ed. (1986);
"Immobilized Cells and Enzymes"
IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996).

Other general references are provided throughout this document. The procedures therein are believed to be well known in the art and are provided for the convenience of the reader.

## MATERIALS AND METHODS

### 5-hmc detection

The T4 β-glucosyltransferase (13-GT) was used to tag 5-hmc sites with a fluorescent reporter molecule. The enzyme was fed with a synthetic cofactor UDP-6-N3-Glu, resulting in covalent attachment of a functional azide at the 5-hmc site. This azide was further reacted with an Cy5 Fluor alkyne via a "dick" chemistry reaction to generate the fluorescently labeled 5-hmc. The resulting DNA product had fluorescence and absorbance proportional to the content of 5-hmc residues. The DNA was stretched on a cover slip glass and visualized by fluorescence microscopy, then all imaged data was analyzed using a proprietary software. The software is counting the number of 5-hmc per length or intensity of the DNA and calculates the percentage of 5-hmc from total DNA bases.

### PBMC isolateion and purification

Fresh blood (30 mL) was diluted with 30 mL of PBS and then carefully layered on top 18 mL Ficoll-paque Plus. The tubes were then centrifuged at 400 X g for 40 min at 20 °C. The plasma layer was removed and discarded, and the PBMC layer was transferred to a fresh 15-mL conical tube and washed 3 times with 10 mL PBS. The cells were resuspended in PBS, and counted.

## DNA extraction

For each sample 300 ul of whole blood (fresh or frozen) was used or $10^6$ of PBMCs. DNA was extracted using the "GenElute-Mammalian Genomic DNA Miniprep Kit" (Sigma). DNA was extracted according to company protocol with one important modification: the samples were never vortexed and wide boar tips were used to pipette the samples in order to maintain long fragments of genomic DNA.

## 5-hmC labelling

DNA (500 ng) was incubated with 2μ! of ß-glycosyltransferase (NEB), 1X buffer 4 (NEB), 20 nM 6-N3-UDPG, and ultra-pure water at 37 °C for overnight. A dick copper-free reaction was used to label the 5-hmC sites with 60 nM DBCO-Cy5 and incubated overnight at 37 °C. DNA samples were cleaned using DNA biding magnetic beads that were purchased from Nvigem (cat# 61001-1500) according to company's protocol. λ bactcriophage genomic DNA (NEB) was used as a negative control since it contains no 5-hmC. No labeling was observed for these samples.

## YoYo-1 staining

DNA was stained with the intercalating dye YOYO-1; 3 μ1 of the sample were diluted in 80 μ1 HEPES -DTT buffer (100uM HEPES, 100uM DTT) with 0.65 μ120 μM YoYo-1 (1:4 dye to nucleotide ratio) and incubated at 37 °C for 2 hours.

## DNA extension

Surfaces **for DNA** extensions were prepared as previously described. In short, 22 x 22 glass cover slips were cleaned for at least 7 hours to overnight by incubation in a freshly made 2:1 (v/v) mixture of 70% nitric acid and 37% hydrochloric acid. After extensive washing with ultrapure water (18 MS2) and then with ethanol, cover slips were dried under a stream of nitrogen. Dry slides were immersed in a premixed solution containing 595 μL N-trimethoxysilylpropyl-N,N,N-trimethylammoniuin chloride and 216 μL of vinyltrimethoxysilane in 300 mL ultrapure water and incubated overnight at 65 °C. After incubation, slides were thoroughly washed with ultrapure water and ethanol and stored at 4 °C in ethanol. The silane solution was freshly made and thoroughly mixed before the slides were introduced into the mixture. Stored slides were normally used within 2 weeks.

## Data acquisition

DNA molecules were extended on silanized glass slides by placing 6 μ1_, of pre-labeled DNA in HEPES -DTT buffer (100uM HEPES, 100uM DTT) between a dry silanized glass slide and a non-treated microscope slide (the DNA final concentration 0.25 ng/μL). Extended DNA molecules were imaged on a MORE imaging system (TILL photonics GmbH) with an Olympus UPlanApo 100X 1.3 NA oil immersion objective. A 150 W Xenon lamp with galvanometer driven filter switching was used as an excitation source. **The filter sets used** to image YOYO-1 stained DNA and the Cy5 labels were 485/20 and 650/13 bandpass excitation filters, 525/30 and 684/24 emission filters. Images were acquired by a DU888 EMCCD (Andor Technologies) with an EM gain setting of 300 and integration times of 200 ms and 3000 ms for YOYO-1 and Cy5, respectively. **All imaged** data was analyzed using a proprietary software. The software is counting the number of 5-hmc per length or intensity of the DNA and calculates the percentage of 5-hmc from total DNA bases.

## EXAMPLE 1

## DETECTION OF 5-HMC IN SOFT TISSUE CANCERS

The 5-hmc level in peripheral blood is estimated to be 0.002 % of total DNA

bases. Existing commercial methods rely on bulky antibodies and cannot detect lower than 0.02 % 5-hmc. More sensitive assays that rely on mass-spectra analysis or radioactive labeling are not suitable for clinical settings. Two commercially available examples are the MethylFlash Hydroxymethylated DNA 5-hmC Quantification Kit (Epigentek cat# p-1036-48) and Quest 5-hmCTM DNA ELISA Kit (Zymo cat# D5425) which are not able to detect 5-hmc in blood. The 5-hmc level of HELA, HEK293 and U2OS cell lines is considered to be extremely low and estimated between 0.001-0.003% of total bases. Herein an imaging method is employed for detecting the level of 5-hmc which is of unprecedented sensitivity (see Figures 1 and 4). Figure 1 shows the wide dynamic range of the methods as described herein supporting its used for all biologically relevant levels of 5hC from healthy brain to blood cancer and cell lines.

In Figure 2 the detection of 5-hmc level in those cell lines is shown and compared to PBMCs of healthy individuals. The detection of as low as 0.001 % or even lower levels of 5-hmc is demonstrated with sufficient precision to compare between such low level 5-hmc contents. It has been shown that 5-hmc level is reduced in cancer tissues (Haffner Oncotarget 2011; 2: 627 - 637) however due to the limitations in sensitivity of existing methods it was impossible to detect the variations of 5-hmc level when comparing peripheral blood of cancer patients and healthy individuals. The results presented herein directly compare the global level of 5-hmc of multiple myeloma and leukemia patients to healthy individuals in their peripheral blood. The level of 5-hmc is dropping on average of 25 % when comparing multiple myeloma patients to healthy individuals (Figure 3). The same results were achieved when comparing leukemia patients to healthy individuals (Figure 3).

**EXAMPLE 2**

DETECTION OF 5-HMC **IN COLORECTA CANCER AND** PRE-MALIGNANT

**REGIONS**

The 5-hmC level of healthy colon in estimated to be 0.1%-0.05% from total **DNA** nucleotides and can drop up to 5 time fold in colon cancer tissue.

Several specific antibodies to 5-hmC are commercially available and can be used for dot blot, immunoprecipitation, and ELISA assays. The detection limit of these commercially available kits is limited to about 0.03 % 5-hmC. However, the detection and screening of colon cancer using those kits is very limited due to poor sensitivity and poor resolution. In most cases the drop of 5-hmC in colon cancer tissues is relatively small (less than 30% drop) and cannot be distinguished when comparing to healthy tissue, if using antibodies related methods. Moreover, in adjacent tissue (healthy tissue next to the tumor) or pre-malignant tissue, the drop of 5-hmC can be as lower than 1 5% compared to a healthy tissue and therefore is undetectable using existent methods.

A chemo-enzymatic labelling scheme is used herein in order to develop a high throughput assay for quantifying the levels of 5-hmC for cancer diagnostics and prediction of treatment outcome. Thus embodiments of the invention rely on fluorescent labelling of 5-hmC followed by fluorescent measurement in a **multi-well slide format** and in single-molecule assay. 5-hmC detection and quantification is based on specific attachment of a fluorescent reporter to individual 5-hmC sites along the DNA molecules.

**Results**

In total 30 commercial colon samples were tested, 10 biopsies form healthy individuals, 10 biopsies of colon cancer patients and 10 biopsies of adjacent tissue,

taken from the same patients. The **DNA** was extracted using commercial available kit, subjected to a chemo-enzymatic reaction and imaged using two different methodologies. Both methods are described in the Materials and Methods section hereinabove. Results show a significant decrease in 5-hmC level in tumor patients vs. healthy individuals. In addition, 5-hmC levels in adjacent healthy tissues were higher than

tumor samples from the same patients, but significantly lower than the level in healthy individuals (Figure 5), attesting to the relevance of the methods described herein for detecting pre-malignant tissues and disease staging. Although the invention has been described in conjunction with specific embodiments thereof, it is evident that many alternatives, modifications and variations will be apparent to those skilled in the art.

**Claims**

1. A method
for detecting and quantifying the level of 5-hydroxymethylcytosine (5-hmc) in a cell-free DNA molecule of a subject using an imaging method, the method comprising:

    associating a label with the 5-hydroxylmethylcytosine, wherein the label comprises a fluorescent label,
    attaching to the DNA molecule an additional labelling agent distinct of the 5-hmc specific label, wherein the additional labelling agent is a non-specific fluorescent label for determining overall DNA fluorescence intensity,
    and
    quantifying the ratio between overall DNA fluorescence intensity and overall 5hmC fluorescence intensity, thereby determining the level of 5-hmc in the cell-free DNA molecule, wherein the method is capable of detecting the level of 5-hmc in a DNA molecule of a cell having a 5-hmc prevalence lower than 0.002 % of total DNA.

2. The method of claim 1, wherein the detecting comprises identifying a position of 5-hydroxymethylcytosine on the cell-free DNA molecule.

3. The method of claim 1, further comprising extracting the cell-free DNA molecule from a sample of the subject.

4. The method of any one of the preceding claims, further comprising comparing the level of 5-hydroxymethylcytosine to a level of 5-hydroxymethylcytosine in a control sample.

5. The method of claim 4, wherein a decrease of the level of 5-hydroxymethylcytosine as compared to the level of 5-hydroxymethylcytosine in the control sample, is indicative of the presence of cancer or a premalignant lesion.

6. The method of claim 4, wherein the control sample is from a healthy subject.

7. The method of claim 5, wherein the method diagnoses cancer in the subject, and wherein the cancer comprises a colon cancer, a pancreatic cancer, an ovarian cancer, a uterine cancer, a lung cancer, a breast cancer, or a prostate cancer.

8. The method of claim 5, wherein the method diagnoses a pre-malignant lesion in the subject.

**9.** The method of claim 1, wherein the label comprises an azide group.

**10.** The method of claim 1, further comprising adding a glucose to the 5-hydroxymethylcytosine.

**11.** The method of claim 10, wherein the glucose is a uridine disphosphoglucose (UDP-Glu).

## Patentansprüche

**1.** Verfahren zum Nachweis und zur Quantifizierung des Gehalts an 5-Hydroxymethylcytosin (5-hmc) in einem zellfreien DNA-Molekül eines Subjekts unter Verwendung eines bildgebenden Verfahrens, wobei das Verfahren umfasst:

Zuordnen einer Markierung zu dem 5-Hydroxymethylcytosin, wobei die Markierung eine fluoreszierende Markierung umfasst,
Anheften eines zusätzlichen Markierungsmittels an das DNA-Molekül, das sich von der 5-hmc-spezifischen Markierung unterscheidet, wobei das zusätzliche Markierungsmittel eine unspezifische fluoreszierende Markierung zur Bestimmung der Gesamt-DNA-Fluoreszenzintensität ist, und
Quantifizieren des Verhältnisses zwischen der Gesamt-DNA-Fluoreszenzintensität und der Gesamt-5hmC-Fluoreszenzintensität, wodurch der Gehalt an 5-hmc in dem zellfreien DNA-Molekül bestimmt wird,
wobei das Verfahren in der Lage ist, den Gehalt an 5-hmc in einem DNA-Molekül einer Zelle mit einer 5-hmc-Prävalenz von weniger als 0,002 % der Gesamt-DNA nachzuweisen.

**2.** Verfahren nach Anspruch 1, wobei der Nachweis das Identifizieren einer Position von 5-Hydroxymethylcytosin auf dem zellfreien DNA-Molekül umfasst.

**3.** Verfahren nach Anspruch 1, das ferner das Extrahieren des zellfreien DNA-Moleküls aus einer Probe des Subjekts umfasst.

**4.** Verfahren nach einem der vorhergehenden Ansprüche, das ferner das Vergleichen des 5-Hydroxymethylcytosingehalts mit einem 5-Hydroxymethylcytosingehalt in einer Kontrollprobe umfasst.

**5.** Verfahren nach Anspruch 4, bei dem eine Abnahme des 5-Hydroxymethylcytosingehalts im Vergleich zum 5-Hydroxymethylcytosingehalt in der Kontrollprobe auf das Vorliegen von Krebs oder einer prämalignen Läsion hinweist.

**6.** Verfahren nach Anspruch 4, bei dem die Kontrollprobe von einem gesunden Subjekt stammt.

**7.** Verfahren nach Anspruch 5, wobei das Verfahren Krebs bei dem Subjekt diagnostiziert und wobei der Krebs einen Dickdarmkrebs, einen Bauchspeicheldrüsenkrebs, einen Eierstockkrebs, einen Gebärmutterkrebs, einen Lungenkrebs, einen Brustkrebs oder einen Prostatakrebs umfasst.

**8.** Verfahren nach Anspruch 5, wobei das Verfahren eine prämaligne Läsion bei dem Subjekt diagnostiziert.

**9.** Verfahren nach Anspruch 1, bei dem die Markierung eine Azidgruppe umfasst.

**10.** Verfahren nach Anspruch 1, das ferner das Zugeben einer Glucose zu dem 5-Hydroxymethylcytosin umfasst.

**11.** Verfahren nach Anspruch 10, bei dem die Glucose eine Uridindiphosphoglucose (UDP-Glu) ist.

## Revendications

**1.** Procédé de détection et de quantification du taux de 5-hydroxyméthylcytosine (5-hmc) dans une molécule d'ADN acellulaire d'un sujet en utilisant un procédé d'imagerie, le procédé comprenant :

l'association d'un marqueur à la 5-hydroxyméthylcytosine, dans lequel le marqueur comprend un marqueur fluorescent,
la fixation à la molécule d'ADN d'un agent de marquage supplémentaire distinct du marqueur spécifique à la 5-hmc, dans lequel l'agent de marquage supplémentaire est un marqueur fluorescent non spécifique pour déterminer l'intensité de fluorescence globale de l'ADN,
et
la quantification du rapport entre l'intensité de fluorescence globale de l'ADN et l'intensité de fluorescence globale de la 5hmC, pour ainsi déterminer le taux de 5-hmc dans la molécule d'ADN acellulaire, dans lequel le procédé est capable de détecter le taux de 5-hmc dans une molécule d'ADN d'une cellule présentant une prévalence de 5-hmc inférieure à 0,002 % de l'ADN total.

**2.** Procédé selon la revendication 1, dans lequel la détection comprend l'identification d'une position de la 5-hydroxyméthylcytosine sur la molécule d'ADN acellulaire.

**3.** Procédé selon la revendication 1, comprenant en

outre l'extraction de la molécule d'ADN acellulaire à partir d'un échantillon du sujet.

**4.** Procédé selon l'une quelconque des revendications précédentes, comprenant en outre la comparaison du taux de 5-hydroxyméthylcytosine à un taux de 5-hydroxyméthylcytosine dans un échantillon témoin.

**5.** Procédé selon la revendication 4, dans lequel une diminution du taux de la 5-hydroxyméthylcytosine par rapport au taux de la 5-hydroxyméthylcytosine dans l'échantillon témoin indique la présence d'un cancer ou d'une lésion prémaligne.

**6.** Procédé selon la revendication 4, dans lequel l'échantillon témoin provient d'un sujet en bonne santé.

**7.** Procédé selon la revendication 5, dans lequel le procédé diagnostique un cancer chez le sujet, et dans lequel le cancer comprend un cancer du côlon, un cancer du pancréas, un cancer de l'ovaire, un cancer de l'utérus, un cancer du poumon, un cancer du sein ou un cancer de la prostate.

**8.** Procédé selon la revendication 5, dans lequel le procédé diagnostique une lésion prémaligne chez le sujet.

**9.** Procédé selon la revendication 1, dans lequel le marqueur comprend un groupe azoture.

**10.** Procédé selon la revendication 1, comprenant en outre l'ajout d'un glucose à la 5-hydroxyméthylcytosine.

**11.** Procédé selon la revendication 10, dans lequel le glucose est un uridine-disphosphoglucose (UDP-Glu).

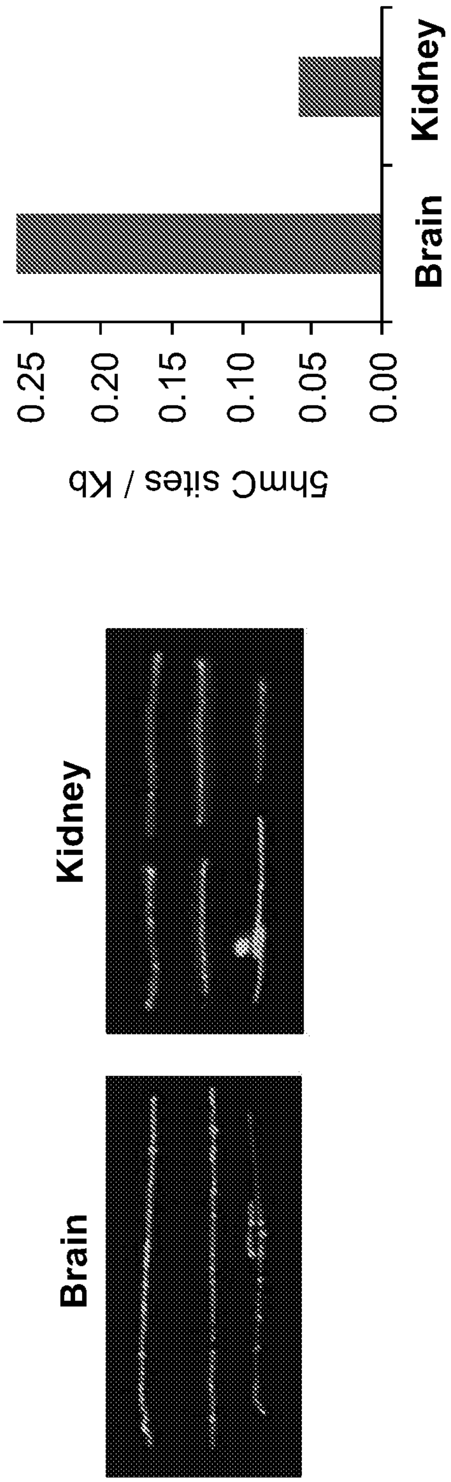
Brain
Kidney
5hmC sites / Kb
0.25
0.20
0.15
0.10
0.05
0.00
Brain
Kidney

Figure 1

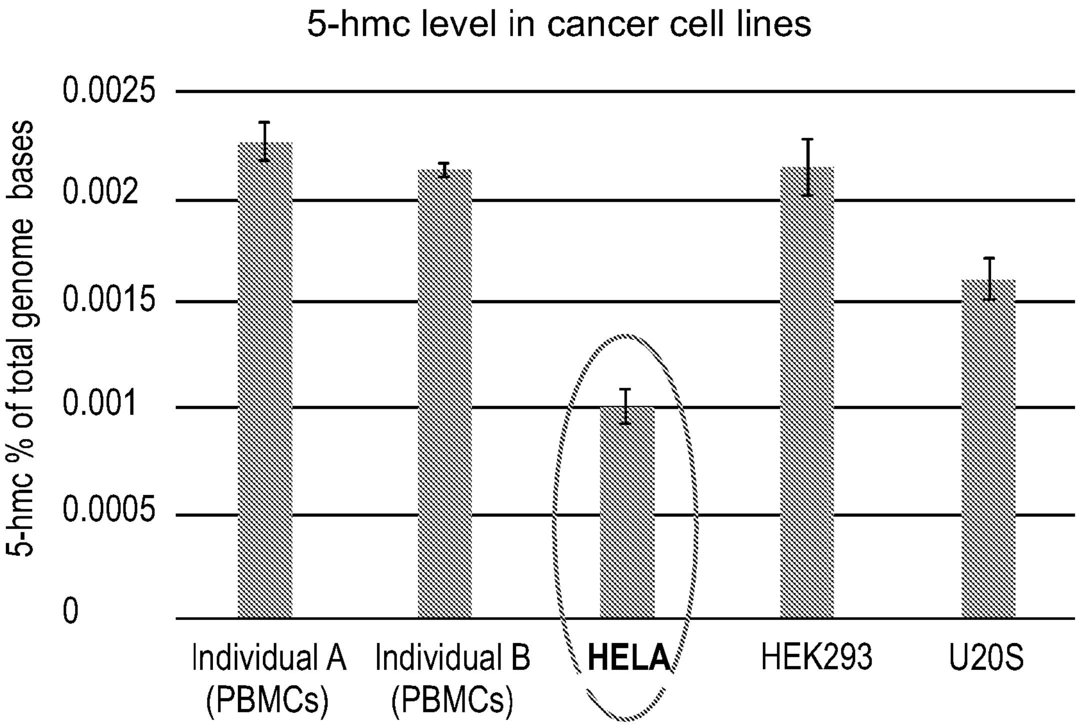
5-hmc level in cancer cell lines
5-hmc % of total genome bases
0.0025
0.002
0.0015
0.001
0.0005
0
Individual A (PBMCs)
Individual B (PBMCs)
HELA
HEK293
U20S

Figure 2

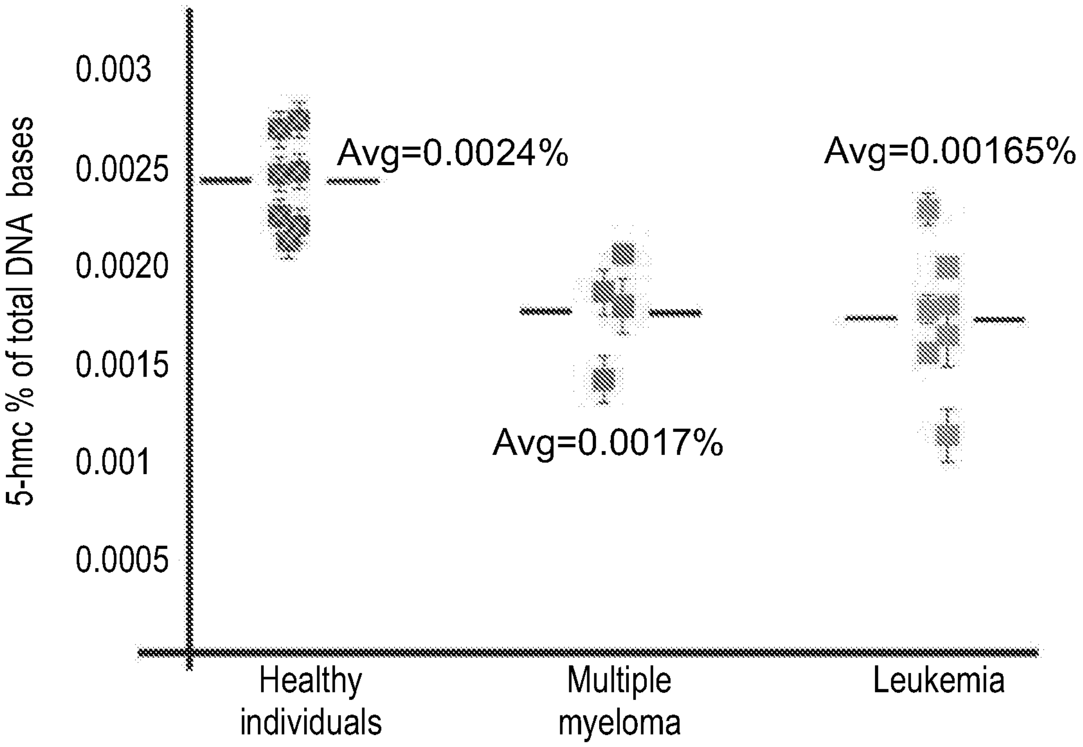

5-hmc % of total DNA bases
0.003
0.0025
0.0020
0.0015
0.001
0.0005
Avg=0.0024%
Avg=0.0017%
Avg=0.00165%
Healthy individuals
Multiple myeloma
Leukemia


Figure 3

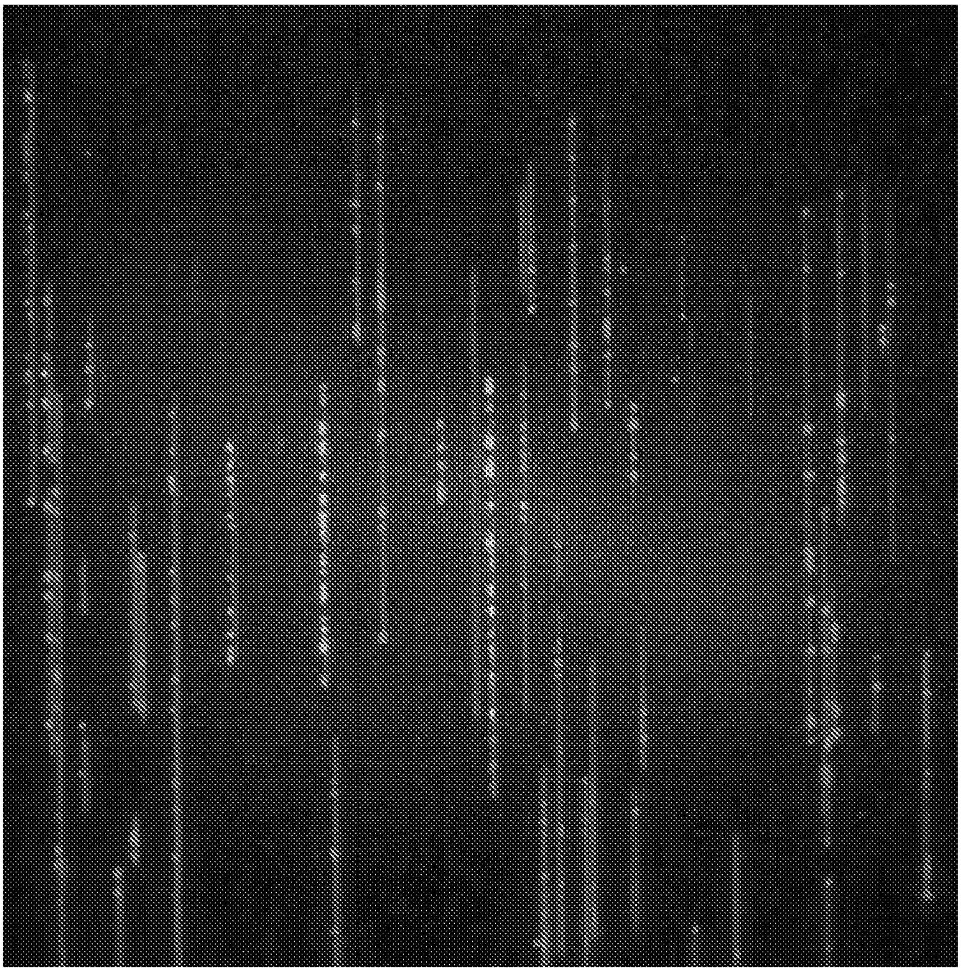

Figure 4

Figure 5A

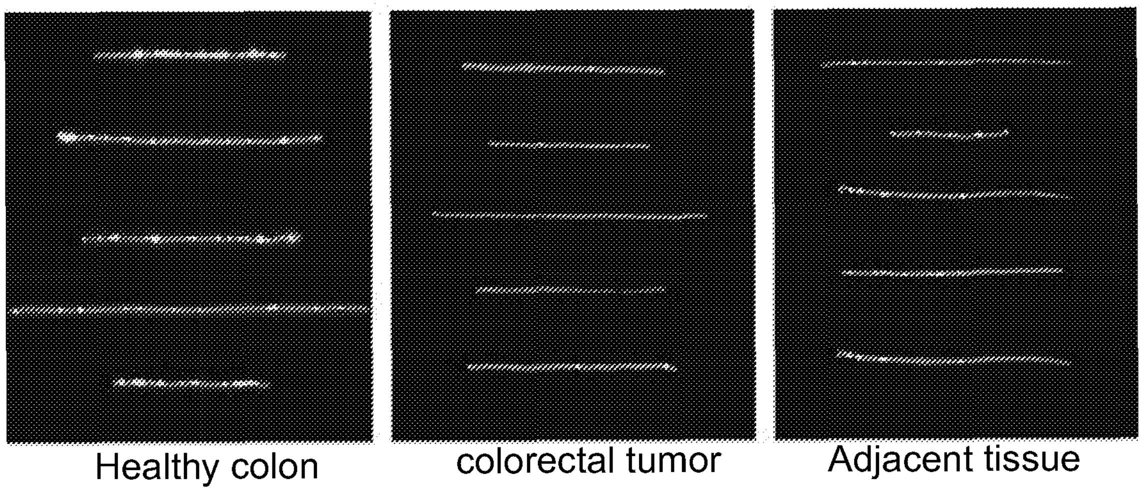


Figure 5B

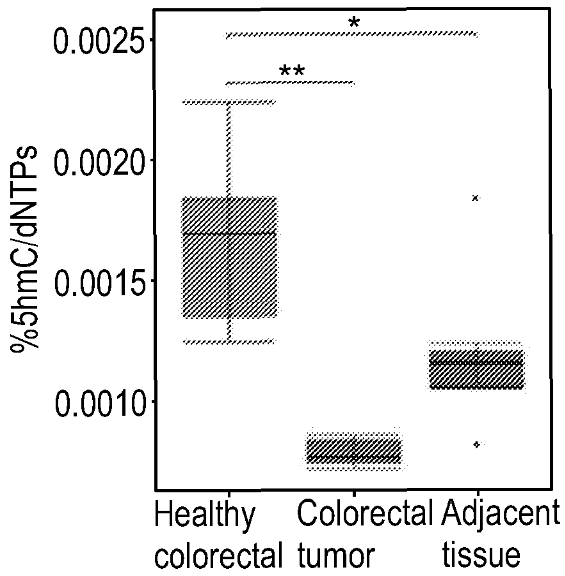

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014191981 A1
- US 2014030727 A1
- WO 2011127136 A, HE CHUAN [US]; SONG CHUNXIAO [US]
- WO 20111013 A
- US 20120122087 A
- WO 2014191981 A
- US 20030124611 A
- US 4666828 A
- US 4683202 A
- US 4801531 A
- US 5192659 A
- US 5272057 A
- US 3791932 A
- US 3839153 A
- US 3850752 A
- US 3850578 A
- US 3853987 A
- US 3867517 A
- US 3879262 A
- US 3901654 A
- US 3935074 A
- US 3984533 A
- US 3996345 A
- US 4034074 A
- US 4098876 A
- US 4879219 A
- US 5011771 A
- US 5281521 A


### Non-patent literature cited in the description


- **FEINBERG A P et al.** *Nature*, 1983, vol. 301, 89-92
- **FINBERG A P et al.** *Cancer Res*, 1988, vol. 48, 159
- **KRIAUCIONIS S et al.** *Science*, 2009
- **TAHILIANI M et al.** *Science*, 2009
- **VALINLUCK V et al.** *Cancer Res*, 2007
- **VALINLUCK V et al.** *Nucleic Acid res*, 2004
- **PENN N W et al.** *Biochem J*, 1976
- **PENN N W et al.** *biochem J*, 1972
- **VALINLUCK V et al.** *Nucleic Acids Res*, 2004
- **LIUTKEVICIUTE Z et al.** *Nat Chem Biol*, 2009
- **ITO S et al.** *Nature*, 2010
- **GIL NIFKER et al.** One-Pot Chemoenzymatic Cascade for Labeling of the Epigenetic Marker 5-Hydroxymethylcytosine. *CHEMBIOCHEM*, 27 July 2015, vol. 16 (13), ISSN 1439-4227, 1857-1860
- **TAMAR SHAHAL et al.** Spectroscopic Quantification of 5-Hydroxymethylcytosine in Genomic DNA. *ANALYTICAL CHEMISTRY*, 29 July 2014, vol. 86, ISSN 0003-2700, 8231-8237
- **YAEL MICHAELI et al.** Optical detection of epigenetic marks: sensitive quantification and direct imaging of individual hydroxymethylcytosine bases. *CHEMICAL COMMUNICATIONS*, 07 October 2013, vol. 49, ISSN 1359-7345, 8599-8720
- **HAFFNER et al.** *Oncotarget*, 2011, vol. 2, 627-637
- **SONG et al.** *Nat. Biotech.*, 2011, vol. 29, 68-72
- **KO et al.** *Immunological Reviews*, vol. 263, 6-21
- **CHEN et al.** *Clinical Chemistry*, 2013, vol. 59, 5
- **YUVAL EBENSTEIN ; DMITRI TORCHINSKY**. Sizing femtogram amounts of dsDNA by single-molecule counting. *Nucl. Acids Res.*, 2016
- *Journal of Nucleic Acids*, vol. 2011
- *Plos one*, December 2010, vol. 5 (12), e15367
- **ZHOU H et al.** *Clinical Chemistry*, 2007
- **V. ROSTOVTSEV ; L. G. GREEN ; V. V. FOKIN ; K. B. SHARPLESS**. *Angew. Chem. Int. Ed.*, 2002, vol. 41, 2596
- **H.C. KOLB ; M. FINN ; K.B. SHARPLESS**. *Angew Chem., Int. Ed.*, 2001, vol. 40, 2004
- **SAMBROOK et al.** Molecular Cloning: A laboratory Manual. 1989
- Current Protocols in Molecular Biology. 1994, vol. I-III
- **AUSUBEL et al.** Current Protocols in Molecular Biology. John Wiley and Sons, 1989
- **PERBAL**. A Practical Guide to Molecular Cloning. John Wiley & Sons, 1988
- **WATSON et al.** Recombinant DNA. Scientific American Books
- Genome Analysis: A Laboratory Manual Series. Cold Spring Harbor Laboratory Press, 1998, vol. 1-4
- Cell Biology: A Laboratory Handbook. 1994, vol. I-III

- Current Protocols in Immunology. 1994, vol. I-III
- Basic and Clinical Immunology. Appleton & Lange, 1994
- Selected Methods in Cellular Immunology. W. H. Freeman and Co., 1980
- Oligonucleotide Synthesis. 1984
- Nucleic Acid Hybridization. 1985
- Transcription and Translation. 1984
- Animal Cell Culture. 1986
- Immobilized Cells and Enzymes. IRL Press, 1986
- **PERBAL, B.** A Practical Guide to Molecular Cloning. 1984
- Methods in Enzymology. Academic Press, vol. 1-317
- PCR Protocols: A Guide To Methods And Applications. Academic Press, 1990
- **MARSHAK et al.** Strategies for Protein Purification and Characterization - A Laboratory Course Manual. CSHL Press, 1996
- **HAFFNER**. *Oncotarget*, 2011, vol. 2, 627-637